# EUROPÄISCHE PATENTSCHRIFT

(11) **EP 1 218 380 B1**
(45) Veröffentlichungstag und Bekanntmachung des Hinweises auf die Patenterteilung: **17.12.2003**
(21) Anmeldenummer: 00964191.1
(22) Anmeldetag: 18.09.2000
(51) Int. Cl.: C07D 487/04, A61K 31/435, A61K 31/495, A61K 31/505, A61K 31/53, A61P 25/00, A61P 43/00

(54) **AM SECHSRING SUBSTITUIERTE, BICYCLISCHE IMIDAZO-3-YL-AMINDERIVATE**
BICYCLIC IMIDAZO-3-YL-AMINE DERIVATIVES WHICH ARE SUBSTITUTED ON THE SIXTH RING
DERIVES D'IMIDAZO-3-YL-AMINE BICYCLIQUES SUBSTITUES SUR LE SIXIEME CHAINON

(30) Priorität: 08.10.1999 DE 19948437; 08.10.1999 DE 19948434
(43) Veröffentlichungstag der Anmeldung: 03.07.2002
(73) Patentinhaber: Grünenthal GmbH, 52078 Aachen (DE)
(72) Erfinder: GERLACH, Matthias, 63636 Brachttal (DE); MAUL, Corinna, 52066 Aachen (DE)
(86) Internationale Anmeldenummer: EP0009095
(87) Internationale Veröffentlichungsnummer: WO01027110

(56) Entgegenhaltungen:
- EP-A- 0 266 890
- EP-A- 0 518 033
- EP-A- 0 822 194
- RIMOLI MG AVALLONE L DE CAPRARIIS P LURASCHI E ABIGNENTE E FILIPPELLI W BERRINO L ROSSI F: "Research on heterocyclic compounds. XXXVII. Synthesis and antiinflammatory activity of methyl-substituted imidazo[1,2-a]pyrazine derivatives" EUROPEAN JOURNAL OF MEDICINAL CHEMISTRY.CHIMICA THERAPEUTICA,FR,EDITIONS SCIENTIFIQUE ELSEVIER, PARIS, Bd. 32, Nr. 3, 1997, Seiten 195-203, XP004075421 ISSN: 0223-5234
- LANERI S ET AL: "Research on heterocyclic compounds - Part XXXIX. 2-Methylimidazo[1,2-a]pyrimidine-3-carboxy lic derivatives: Synthesis and antiinflammatory activity" EUROPEAN JOURNAL OF MEDICINAL CHEMISTRY.CHIMICA THERAPEUTICA,FR,EDITIONS SCIENTIFIQUE ELSEVIER, PARIS, Bd. 33, Nr. 3, 1. März 1998 (1998-03-01), Seiten 163-170, XP004122908 ISSN: 0223-5234
- SAACHI A LANERI S ARENA F LURASCHI E ABIGNENTE E D'AMICO M BERRINO L ROSSI F: "Research on heterocyclic compounds. Part XXXVI. Imidazo[1,2-a]pyrimidine-2-acetic derivatives: synthesis and antiinflammatory activity" EUROPEAN JOURNAL OF MEDICINAL CHEMISTRY.CHIMICA THERAPEUTICA,FR,EDITIONS SCIENTIFIQUE ELSEVIER, PARIS, Bd. 32, Nr. 7-8, 1. Juli 1997 (1997-07-01), Seiten 677-682, XP004088416 ISSN: 0223-5234 in der Anmeldung erwähnt
- BLACKBURN C ET AL: "Parallel Synthesis of 3-Aminoimidazo[1,2-a]pyridines and pyrazines by a New Three-Component Condensation" TETRAHEDRON LETTERS,NL,ELSEVIER SCIENCE PUBLISHERS, AMSTERDAM, Bd. 39, Nr. 22, 28. Mai 1998 (1998-05-28), Seiten 3635-3638, XP004118699 ISSN: 0040-4039 in der Anmeldung erwähnt
- BLACKBURN C: "A Three-Component Solid-Phase Synthesis of 3-Aminoimidazo[1,2-a]azines" TETRAHEDRON LETTERS,NL,ELSEVIER SCIENCE PUBLISHERS, AMSTERDAM, Bd. 39, Nr. 31, 30. Juli 1998 (1998-07-30), Seiten 5469-5472, XP004124091 ISSN: 0040-4039 in der Anmeldung erwähnt
- H.BIENAYME ET AL.: "A New Heterocyclic Multicomponent Reaction for the Combinatorial Synthesis of Fused 3-Aminoimidazoles." ANGEW. CHEM. INT. EDN., Bd. 37, Nr. 16, 1998, Seiten 2234-2237, XP000978871 in der Anmeldung erwähnt

## Beschreibung

Die vorliegende Erfindung betrifft am Sechsring substituierte, bicyclische Imidazo-3-yl-aminderivate und Arzneimittel enthaltend diese Verbindungen.

Für einzelnen Verbindungen aus der Klasse der Imidazo-3-yl-amine sind interessante pharmakologische Eigenschaften bekannt. So werden bestimmte Imidazo[1,2-a]pyridine als den Blutdruck senkende Wirkstoffe (GB-B-1,135,893), als Anthelminthika und Antimykotika (J. Med. Chem. 1972, 15, 982-985)und als antisekretorische Wirkstoffe zur Behandlung von entzündlichen Erkrankungen (EP-A-0 068 378) beschrieben. Eine Wirkung einzelner Imidazopyridine gegen entzündliche Erkrankungen insbesondere des Magens beschreiben auch EP-A-0 266 890 und J. Med. Chem. 1987, 30, 2031-2046. Weitere, für einzelne Vertreter aus der Klasse der Imidazo-3-yl-amine beschriebene pharmakologische Wirkungen sind antibakterielle Eigenschaften (Chem. Pharm. Bull. 1992, 40, 1170), antivirale Eigenschaften (J. Med. Chem. 1998, 41, 5108-5112) sowie die Wirkung als Benzodiazepin-Rezeptor Antagonist (J. Heterocyclic Chem. 1998,35, 1205-1217).

Angesichts dieser interessanten Wirkungen wurden in der Vergangenheit verschiedene Vertreter aus der Klasse der substituierten Imidazo-3-yl-amine synthetisiert. Insbesondere wurde versucht, die Zahl der verfügbaren substiuierten Imidazo-3-yl-amine durch kombinatorische Syntheseverfahren zu vergrößern. So beschreiben C. Blackburn et al. in Tetrahedron Lett. 1998, 39, 5469-5472 eine Dreikomponenten-Festphasensynthese zur Herstellung von Imidazo-3-yl-aminen und in Tetrahedron Lett. 1998, 39, 3635-3638 eine Dreikomponenten-Kondensation zur Parallelsynthese von Imidazo-3-ylaminen. Ähnlich der letztgenannten Reaktion ist die in von K. Groebke et al.in Synlett 1998, 661-663 publizierte Synthese. Eine Mehrkomponentenreaktion für die kombinatorische Synthese von Imidazo-3-yl-aminen, mit der auch vereinzelte Imidazo-5-amine hergestellt wurden, beschreiben auch H. Bienayme und K. Bouzid in Angew. Chem. 1998, 110 (16), 2349-2352.

Die gemäß dem Stand der Technik mögliche Variationsbreite der Substituenten am Amino-Stickstoff und in der 2-Position des Imidazolrings war jedoch begrenzt.

Der vorliegenden Erfindung lag daher die Aufgabe zugrunde, weitere bicyclische Imidazo-3-yl-amine, bei denen mindestens ein Substituent an der Aminogruppe und an den nicht zum Imidazolring gehörenden Atomen des Sechsrings sowie der Substituent in der 2-Position des Imidazolrings von Wasserstoff verschieden sind, und diese enthaltende Arzneimittel bereitzustellen.

Gegenstand der Erfindung sind daher bicyclische Imidazo-3-yl-amine der allgemeinen Formel I, 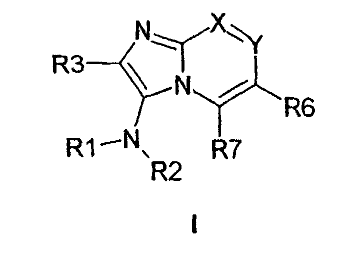 worin
X CR⁴ oder N und Y CR⁵ oder N bedeuten mit der Maßgabe, daß X und Y nicht gleichzeitig N bedeuten,
R¹ (CH₂)ₙCN mit n = 4 ,5 oder 6, gegebenenfalls substituiertes Phenyl, C₄-C₈-Cycloalkyl, CH₂CH₂R (R = 4-Morpholino), 1,1,3,3,-Tetramethylbutyl oder CH₂R^{a}, wobei R^{a} für Wasserstoff, OH, C₁-C₈-Alkyl (verzweigt oder unverzweigt), gegebenenfalls substituiertes Phenyl, CO(OR') (mit R' = unverzweigtes C₁-C₄-Alkyl oder verzweigtes C₁-C₅-Alkyl), PO(OR')₂ (mit R' = unverzweigtes C₁-C₄-Alkyl oder verzweigtes C₁-C₅-Alkyl) oder Si(R^{x}R^{y}R^{z}) (mit R^{x}, R^{y}, und R^{z} jeweils unabhängig voneinander C₁-C₄-Alkyl(verzweigt oder unverzweigt), C₄-C₈-Cycloalkyl oder Phenyl) steht, bedeutet,
R² Wasserstoff, COR^{b}, wobei R^{b} für C₁-C₄-Alkyl(verzweigt oder unverzweigt)oder C₃-C₈-Cycloalkyl steht, CH₂CH₂CO(OR^{c}), wobei R^{c} für C₁-C₄-Alkyl (verzweigt oder unverzweigt), Adamantyl, gegebenenfalls substituiertes Phenyl, gegebenenfalls substituiertes 1-Naphtyl oder 2-Naphtyl oder jeweils gegebenenfalls substiuiertes 2-Pyridyl, 3-Pyridyl, 4-Pyridyl, Thiazolyl oder Furoyl steht, CH₂Phenyl, CH₂CH₂R^{d}, wobei R^{d} für gegebenenfalls substituiertes Phenyl steht, oder CONHR^{e}, wobei R^{e} für C₁-C₈-Alkyl(verzweigt oder unverzweigt), C₃-C₈-Cycloalkyl oder gegebenenfalls substiuiertes Phenyl steht, bedeutet
R³ C₁-C₈-Alkyl(verzweigt oder unverzweigt), C₃-C₈-Cycloalkyl, gegebenenfalls substituiertes Phenyl, gegebenenfalls substituiertes Naphtyl, gegebenenfalls substituiertes Pyrrol, gegebenenfalls substituiertes Pyridyl, gegebenenfalls substituiertes Furan, gegebenenfalls substituiertes Thiophen, gegebenenfalls substituiertes Anthracen, gegebenenfalls substituiertes Phenanthren oder gegebenenfalls substituiertes Chinolin bedeutet,
R⁴, R⁵, R⁶ und R⁷ jeweils unabhängig voneinander Wasserstoff, C₁-C₄-Alkyl(verzweigt oder unverzweigt), NO₂, NH₂, OH, CF₃, Cl, F, Br, CN, COOR, wobei R für Wasserstoff, C₁-C₄-Alkyl(verzweigt oder unverzweigt) steht, OR'', wobei R'' für gegebenenfalls substituiertes Phenyl, gegebenenfalls substituiertes Benzyl, C₁-C₄-Alkyl(verzweigt oder unverzweigt) steht, oder
R⁴ und R⁵, R⁵ und R⁶ oder R⁶ und R⁷ eine viergliedrige, gegebenenfalls Doppelbindungen enthaltende Kohlenstoffbrücke, in der gegebenenfalls einzelne C-Atome durch Heteroatome wie N, O oder S ersetzt sein können, bedeuten und alle darüber hinaus vorhandenen, nicht die Brücke bildenden Reste R⁴, R⁵, R⁶ und R⁷ Wasserstoff bedeuten,
mit der Maßgabe, daß mindestens einer der im Molekül vorhandenen Reste R⁴, R⁵, R⁶ oder R⁷ nicht Wasserstoff ist und, wenn R¹ Methyl, Ethyl , Propyl, n-Butyl, iso-Butyl, CO(OMethyl) oder Benzyl bedeutet, R³ nicht Methyl bedeutet, und, wenn einer der Reste R⁴, R⁵, R⁶ und R⁷ OBenzyl bedeutet oder R⁴ OC₁-C₄-Alkyl(verzweigt oder unverzweigt) bedeutet, R¹ nicht CH R^{a} (wobei R^{a} für Wasserstoff oder C₁-C₅-Alkyl (verzweigt oder unverzweigt) steht) bedeutet , in Form der Basen oder von pharmazeutisch akzeptablen Salzen.

Erfindungsgemaß bevorzugt sind dabei solche Verbindungen, bei denen R² Wasserstoff bedeutet,
R¹ ausgewählt ist aus der Gruppe (CH₂)ₙCN mit n = 4 ,5 oder 6, Cyclohexyl, CH₂CO(OMethyl), 2,6-Dimethylphenyl, 1,1,3,3,-Tetramethylbutyl oder n-Butyl und
R³ ausgewählt ist aus der Gruppe 2-Pyridyl, 3-Pyridyl, 2-Furanyl, 2-Pyrroyl, Methyl, *tert*-Butyl, 3-Hydroxyphenyl, 3,4-Dimethoxyphenyl, 2,3-Dichlorphenyl, 2,4-Dichlorphenyl, 2-Methoxyphenyl, 2,3-Dimethoxyphenyl, 3-Bromphenyl, 4-Brom-2-fluorphenyl, 5-Brom-2-fluorphenyl, 3-Brom-4-fluorphenyl, 3-Chlorphenyl, 3,4-Dichlorphenyl, 3-Fluorphenyl, 3-Methylphenyl, 3-Phenoxyphenyl, 3-(4-Chlorphenoxy)phenyl, 2-Chlor-4-fluorphenyl, 2-Chlor-6-fluorphenyl, 2,4-Dimethylphenyl, 2,5-Dimethylphenyl, 2-Bromphenyl, 2-Fluorphenyl, 2-(Trifluormethyl)-phenyl.

Die Reste R⁴, R⁵, R⁶ und R⁷ sind erfindungsgemäß bevorzugt entweder ausgewählt aus der Gruppe Wasserstoff, NO₂, NH₂, OH, CF₃, Cl, F, Br, CN, Methyl oder OR" mit R'' = Benzyl, wobei mindestens einer der Reste R⁴, R⁵, R⁶ und R⁷ von Wasserstoff verschieden sein muß, oder R⁶ und R⁷ bilden gemeinsam eine Brücke -CH=CH-CH=CH- und die Reste R⁴ und R⁵, soweit vorhanden, bedeuten Wasserstoff.

Besonders bevorzugt sind erfindungsgemäß am Sechsring substituierte, bicyclische Imidazo-3-yl-aminderivate ausgewählt aus der Gruppe
7-Chlor-2-furan-2-yl-(6-isocyanohexyl)-imidazo[1,2-a]pyrimidin-3,5-diamin,
(5,7-Dimethyl-2-pyridin-2-yl-imidazo[1,2-a]pyrimidin-3-yl)-(6-isocyano-hexyl)-amin,
7-Chlor-(1,1,3,3-tetramethylbutyl)-2-thiophen-2-ylimidazo[1,2-a]pyrimidin-3,5-diamin,
[6-Brom-2-(2-methoxyphenyl)-imidazo[1,2-a]pyridin-3-yl]-(1,1,3,3-tetramethylbutyl)-amin,
*N*-[4-(8-Benzyloxy-3-cyclohexylamino-imidazo[1,2-a]pyridin-2-yl)-phenyl]-acetamid,
3-(8-Benzyloxy-3-butylamino-imidazo[1,2-a]pyridin-2-yl)-phenol,
[8-Benzyloxy-2-(3,5-dimethoxyphenyl)-imidazo[1,2-a]pyridin-3-ylamino]-essigsäuremethylester,
[8-Benzyloxy-2-(3,5-dimethoxy-phenyl)-imidazo[1,2-a]pyridin-3-yl]-cyclohexylamin,
Cyclohexyl-[6,8-dibrom-2-(2-methoxy-phenyl)-5-methylimidazo[1,2-a]pyridin-3-yl]-amin,
3-[3-(2,6-Dimethylphenylamino)-6-nitro-imidazo[1,2-a)pyridin-2-yl]-phenol,
[6-Brom-2-(2-methoxyphenyl)-imidazo[1,2-a]pyridin-3-yl]-(1,1,3,3-tetramethylbutyl)-amin,
[6,8-Dibrom-2-(2,3-dimethoxyphenyl)-5-methylimidazo[1,2-a]pyridin-3-yl]-(1,1,3,3-tetramethylbutyl)-amin,
Cyclohexyl-(2-phenyl-imidazo[1,2-a]chinolin-1-yl)-amin.
[6-(2-Cyclohexyl-5-methyl-imidazo[1,2-a]pyridin-3-ylamino)-hexyl]-methylidyn-ammonium,
(2, 6-Dimethyl-imidazo[1,2-a]pyridin-3-yl)-(1, 1,3, 3-tetramethylbutyl)-amin,
Cyclohexyl-(2,7-dimethyl-imidazo[1,2-a]pyridin-3-yl)-amin,
Cyclohexyl- (2, 5, 7-trimethyl-imidazo[1,2-a]pyridin-3-yl)-amin,
[2-(3,4-Dimethoxyphenyl)-6-methyl-imidazo[1,2-a]pyridin-3-yl]-(6-isocyanohexyl)-amin,
(2, 7-Dimethyl-imidazo [1,2-a]pyridin-3-yl)-(1,1, 3, 3-tetramethyl-butyl)-amin,
(2,8-Dimethyl-imidazo[1,2-a]pyridin-3-yl)-(1, 1, 3, 3-tetramethyl-butyl)-amin
(1,1,3,3-Tetramethyl-butyl)-(2,5,7-trimethylimidazo[1,2-a]pyridin-3-yl)-amin,
[2-(3,4-Dimethoxy-phenyl)-7-methyl-imidazo[1,2-a]pyridin-3-yl]-(6-isocyano-hexyl)-amin,
(6-Isocyano-hexyl)-[2-(2-methoxy-phenyl)-6-methylimidazo[1,2-a]pyridin-3-yl]-amin,
Cyclohexyl-(2-furan-2-yl-6-trifluoromethyl-imidazo[1,2-a]pyridin-3-yl)-amin;
(8-Benzyloxy-2-cyclohexyl-imidazo[1,2-a]pyridin-3-yl)-cyclohexyl-amin,
(8-Benzyloxy-2-methyl-imidazo[1,2-a]pyridin-3-yl)-cyclohexyl-amin,
(8-Benzyloxy-2-methyl-imidazo[1,2-a]pyridin-3-yl)-(1,1,3,3-tetramethyl-butyl)-amin,
(8-Benzyloxy-2-cyclohexyl-imidazo [1,2-a]pyridin-3-ylamino)-essigsäuremethylester,
(8-Benzyloxy-2-methyl-imidazo[1,2-a]pyridin-3-ylamino)-essigsäuremethylester,
Butyl-(2-cyclohexyl-5-propyl-imidazo[1,2-a]pyridin-3-yl)-amin,
N-Cyclohexyl-N-(6,8-dichlor-2-furan-2-yl-imidazo[1,2-a]pyridin-3-yl)-acetamid,
N-Cyclohexyl-N-(2-furan-2-yl-6-trifluormethylimidazo[1,2-a]pyridin-3-yl)-acetamid,
N-(8-Benzyloxy-2-cyclohexyl-imidazo[1,2-a]pyridin-3-yl)-N-cyclohexyl-acetamid,
(5-Methyl-2-phenanthren-9-yl-imidazo[1,2-a]pyridin-3-yl)-(1,1,3,3-tetramethyl-butyl)-amin,
(2-Anthracen-9-yl-7-methyl-imidazo[1,2-a]pyrimidin-3-yl)-(1,2,3,3-tetramethyl-butyl)-amin oder
Cyclohexyl-[7-methyl-2-(1-methyl-1H-pyrrol-2-yl)-imidazo[1,2-a]pyrimidin-3-yl]-amin.

Soweit die erfindungsgemäßen am Sechsring substituierten, bicyclischen Imidazo-3-yl-aminderivate optisch aktive Kohlenstoffatome enthalten, sind auch die Enantiomeren dieser Verbindungen und deren Mischungen Gegenstand der vorliegenden Erfindung.

Gegenstand der Erfindung sind außerdem Arzneimittel enthaltend als Wirkstoff mindestens ein bicyclisches Imidazo-3-yl-amin der allgemeinen Formel I, in der R¹ bis R⁷, X und Y die oben angegebene Bedeutung haben, in Form der Base oder eines pharmazeutisch akzeptablen Salzes, vorzugsweise der Bromwasserstoffsäure, Schwefelsäure, Methansulfonsäure, Ameisensäure, Essigsäure, Oxalsäure, Bernsteinsäure, Weinsäure, Mandelsäure, Furmarsäure, Milchsäure, Zitronensäure, Glutaminsäure und/ oder Asparaginsäure oder insbesondere der Salzsäure.

Überraschenderweise wurde dabei gefunden, daß die erfindungsgemäßen Verbindungen nicht nur potentielle Wirkstoffe für die im Stand der Technik genannten Indikationen sind, sondern auch analgetische Wirkung zeigen.

Besonders bevorzugt enthalten die erfindungsgemäßenen Arzneimittel als Wirkstoff mindestens ein bicyclisches Imidazo-3-yl-amin ausgewählt aus der Gruppe
7-Chlor-2-furan-2-yl-(6-isocyanohexyl)-imidazo[1,2-a]pyrimidin-3,5-diamin,
(5,7-Dimethyl-2-pyridin-2-yl-imidazo[1,2-a]pyrimidin-3-yl)-(6-isocyano-hexyl)-amin,
7-Chlor-(1,1,3,3-tetramethylbutyl)-2-thiophen-2-yl-imidazo[1,2-a]pyrimidin-3,5-diamin,
[6-Brom-2-(2-methoxyphenyl)-imidazo[1,2-a]pyridin-3-yl]-(1,1,3,3-tetramethylbutyl)-amin,
*N*-[4-(8-Benzyloxy-3-cyclohexylamino-imidazo[1,2-a]pyridin-2-yl)-phenyl]-acetamid,
3-(8-Benzyloxy-3-butylamino-imidazo[1,2-a]pyridin-2-yl)-phenol,
[8-Benzyloxy-2-(3,5-dimethoxyphenyl)-imidazo[1,2-a]pyridin-3-ylamino]-essigsäuremethylester,
[8-Benzyloxy-2-(3,5-dimethoxyphenyl)-imidazo[1,2-a]pyridin-3-yl]-cyclohexyl-amin,
Cyclohexyl-[6,8-dibrom-2-(2-methoxyphenyl)-5-methylimidazo[1,2-a]pyridin-3-yl]-amin,
3- [3- (2, 6-Dimethylphenylamino)-6-nitro-imidazo[1,2-a]pyridin-2-yl]-phenol,
[6-Brom-2-(2-methoxyphenyl)-imidazo[1,2-a]pyridin-3-yl]-(1,1,3,3-tetramethylbutyl)-amin,
[6,8-Dibrom-2-(2,3-dimethoxyphenyl)-5-methylimidazo[1,2-a]pyridin-3-yl]-(1,1,3,3-tetramethylbutyl)-amin oder
Cyclohexyl-(2-phenyl-imidazo[1,2-a]chinolin-1-yl)-amin,
[6-(2-Cyclohexyl-5-methyl-imidazo[2,2-a]pyridin-3-ylamino)-hexyl]-methylidyn-ammonium,
(2,6-Dimethyl-imidazo[1,2-a]pyridin-3-yl)-(1,1,3, 3-tetramethylbutyl)-amin,
Cyclohexyl-(2,7-dimethyl-imidazo[1,2-a]pyridin-3-yl)-amin,
Cyclohexyl-(2,5,7-trimethyl-imidazo[1,2-a]pyridin-3-yl)-amin,
[2-(3,4-Dimethoxyphenyl)-6-methyl-imidazo[1,2-a]pyridin-3-yl]-(6-isocyanohexyl)-amin,
(2,7-Dimethyl-imidazo[1,2-a]pyridin-3-yl)-(1,1,3,3-tetramethyl-butyl)-amin,
(2,8-Dimethyl-imidazo[1,2-a]pyridin-3-yl)-(1,1,3,3-tetramethyl-butyl)-amin
(1,1,3,3-Tetramethyl-butyl)-(2,5,7-trimethylimidazo[1,2-a]pyridin-3-yl)-amin,
[2-(3,4-Dimethoxy-phenyl)-7-methyl-imidazo[1,2-a]pyridin-3-yl]-(6-isocyano-hexyl)-amin,
(6-Isocyano-hexyl)-[2-(2-methoxy-phenyl)-6-methylimidazo[1,2-a]pyridin-3-yl]-amin,
Cyclohexyl-(2-furan-2-yl-6-trifluoromethyl-imidazo[1,2-a]pyridin-3-yl)-amin;
(8-Benzyloxy-2-cyclohexyl-imidazo[1,2-a]pyridin-3-yl)-cyclohexyl-amin,
(8-Benzyloxy-2-methyl-imidazo[1,2-a]pyridin-3-yl)-cyclohexyl-amin,
(8-Benzyloxy-2-methyl-imidazo[1,2-a]pyridin-3-yl)-(1,1,3,3-tetramethyl-butyl)-amin,
(8-Benzyloxy-2-cyclohexyl-imidazo[1,2-a]pyridin-3-ylamino)-essigsäuremethylester,
(8-Benzyloxy-2-methyl-imidazo[1,2-a]pyridin-3-ylamino)-essigsäuremethylester,
Butyl-(2-cyclohexyl-5-propyl-imidazo[1,2-a]pyridin-3-yl)-amin,
N-Cyclohexyl-N-(6,8-dichlor-2-furan-2-yl-imidazo[1,2-a]pyridin-3-yl)-acetamid,
N-Cyclohexyl-N-(2-furan-2-yl-6-trifluormethylimidazo[1,2-a]pyridin-3-yl)-acetamid,
N-(8-Benzyloxy-2-cyclohexyl-imidazo[1,2-a]pyridin-3-yl)-N-cyclohexyl-acetamid,
(5-Methyl-2-phenanthren-9-yl-imidazo[1,2-a]pyridin-3-yl)-(1,1,3,3-tetramethyl-butyl)-amin,
(2-Anthracen-9-yl-7-methyl-imidazo[1,2-a]pyrimidin-3-yl)-(1,1,3,3-tetramethyl-butyl)-amin,
Cyclohexyl-[7-methyl-2-(2-methyl-1H-pyrrol-2-yl)-imidazo[1,2-a]pyrimidin-3-yl]-amin oder der pharmazeutisch akzeptablen Salze dieser Verbindungen.

Die erfindungsgemäßen Verbindungen erwiesen sich als Liganden des schmerzrelevanten α2-Subtyps des humanen α-adrenergen Rezeptors. Besonders bevorzugt ist daher die Verwendung der erfindungsgemäßen bicyclischen Imidazo-5-yl-aminderivate zusammen mit einem oder mehreren Hilfsstoffen zur Herstellung eines Arzneimittels zur Bekämpfung von Schmerz.

Zur Herstellung entsprechender Arzneimittel werden neben mindestens einem erfindungsgemäßen Wirkstoff Trägermaterialien, Füllstoffe, Lösungsmittel, Verdünnungsmittel, Farbstoffe und/oder Bindemittel eingesetzt. Die Auswahl der Hilfsstoffe sowie die einzusetzenden Mengen derselben hängt davon ab, ob das Arzneimittel oral, intravenös, intraperitoneal, intradermal, intramuskulär, intranasal, buccal oder örtlich appliziert werden soll. Für orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße Wirkstoffe in einem Depot, in gelöster Form oder in einem Pflaster, gegebenenfalls unter Zusatz von die Hautpenetration fördernden Mittel, sind geeignete perkutane Applikationszubereitungen. Oral oder perkutan anwendbare Zubereitungsformen können die erfindungsgemäßen Wirkstoffe verzögert freisetzen.

Die an den Patienten zu verabreichende Wirkstoffmenge variiert in Abhängigkeit vom Gewicht des Patienten, von der Applikationsart, der Indikation und dem Schweregrad der Erkrankung.

Die Synthese der erfindungsgemäßen Verbindungen erfolgt in der Weise, daß man Amidine mit der allgemeinen Formel **II**, insbesondere 2-Aminopyridin- und 2-Aminopyrimidinderivate, die von Firmen wie beispielsweise Acros, Avocado, Aldrich, Fluka, Lancaster, Maybridge, Merck, Sigma oder TCI-Jp kommerziell angeboten werden, mit verschiedensten Ketonen oder vorzugsweise Aldehyden **III** und Isonitrilen **IV** in Gegenwart von 20%-iger Perchlorsäure gemäß einer Dreikomponentenraktion umsetzt. R¹ bis R⁷, X und Y haben dabei die oben für Verbindungen der Formel **I** angegebene Bedeutung.

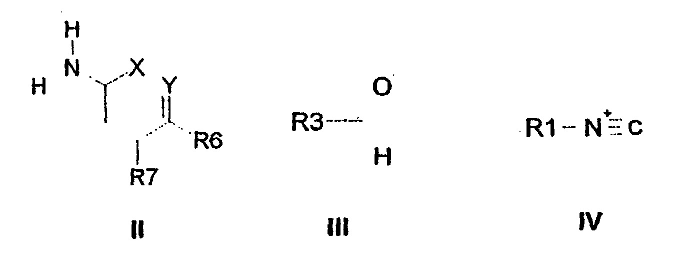

Für einen problemlosen Ablauf der Reaktion ist es dabei wesentlich, daß die Ausgangsverbindungen nacheinander in der Reihenfolge Amidin **II**, Keton oder Aldehyd **III** und Isonitril **IV** zugegeben werden. Vorzugsweise werden die Reaktionen in Dichlormethan bei einer Temperatur von vorzugsweise 0°C bis 40°C, insbesondere bei einer Temperatur von 10°C bis 20°C durchgeführt.

Zur Herstellung der erfindungsgemäßen Verbindungen, in denen R² nicht Wasserstoff bedeutet, werden die in der zuvor beschriebenen Reaktion entstehenden Verbindungen Ia, die vorzugsweise zunächst in THF gelöst wurden, je nach gewünschtem Endprodukt mit einer Verbindung R²Hal, wobei Hal für Brom, Iod oder insbesondere Chlor steht, beispielsweise einem gegebenenfalls substituierten Alkyl-, Aryl- oder Säurechlorid, oder einem gegebenenfalls substituierten Isocyanat R^{e}NCO in Gegenwart eines Morpholin-Harzes (z.B. Polystyrol-Morpholin der Firma Argonaut) in Dichlormethan innerhalb von 0,25 bis 24 Stunden bei Temperaturen zwischen 10°C und 40°C gemäß dem folgenden Reaktionsschema umgesetzt:

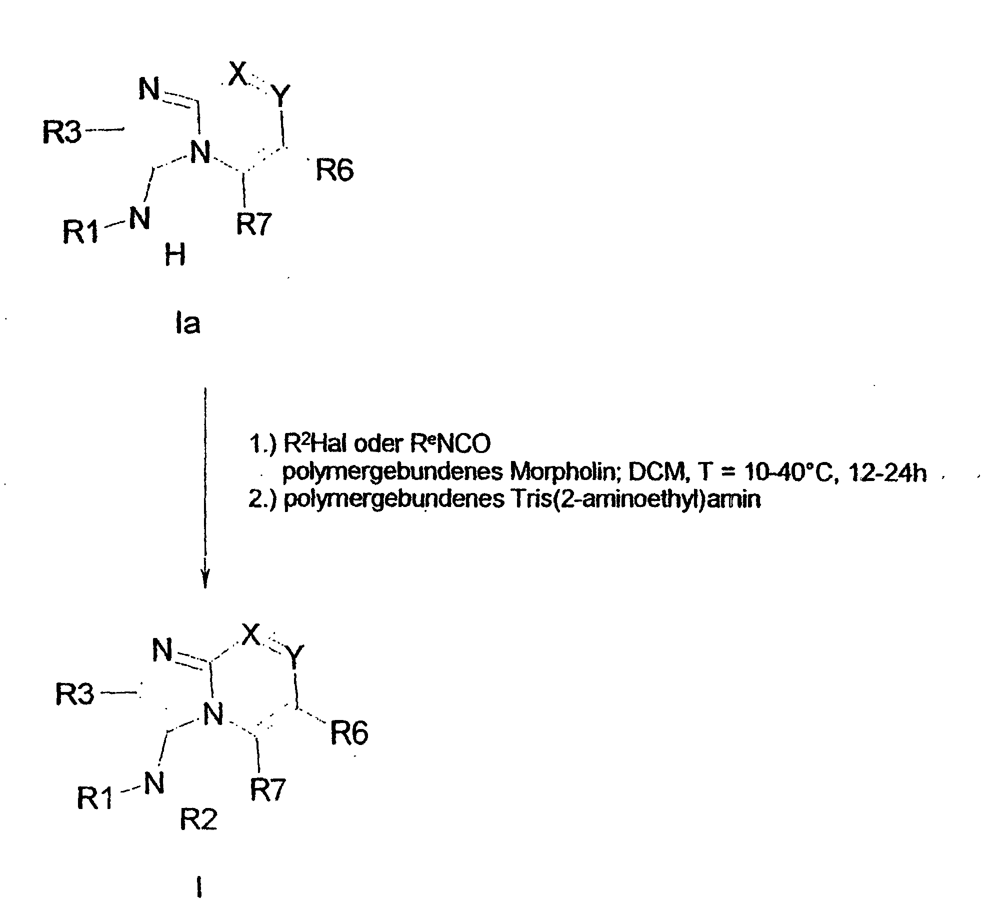

Die überschüssigen Reagentien werden anschließend durch Filtration über eine Schicht mit polymergebundenem Tris(2-aminoethyl)amin (Hersteller: Novabiochem) oder 3-(3-Mercaptophenyl)propanamidomethylpolystyrol aus dem Reaktionsgemisch entfernt und das Filtrat vorzugsweise in einer Vakuumzentrifuge aufkonzentriert. Das gesamte Verfahren läßt sich ohne weiteres auch in einer automatisierten Syntheseanlage durchführen.

Die Verbindungen der Formel **I** lassen sich mit physiologisch verträglichen Säuren, vorzugsweise Bromwasserstoffsäure, Schwefelsäure, Methansulfonsäure, Ameisensäure, Essigsäure, Oxalsäure, Bernsteinsäure, Weinsäure, Mandelsäure, Furmarsäure, Milchsäure, Zitronensäure, Glutaminsäure und/ oder Asparaginsäure und insbesondere Salzsäure, in der an sich bekannten Weise in ihre Salze überführen. Vorzugsweise wird die Salzbildung in einem Lösungsmittel, insbesondere Diethylether, Diisopropylether, Essigsäurealkylester, Aceton oder 2-Butanon oder einem Gemisch dieser Lösungsmittel durchgeführt. Zur Herstellung der Hydrochloride eignet sich alternativ auch Trimethylsilan in wässriger Lösung.

### Beispiele:

Die folgenden Beispiele sollen die Erfindung erläutern, ohne sie darauf zu beschränken.

Die Synthese der erfolgte auf einer automatischen Anlage der Firma Zymark nach folgender allgemeiner Synthesevorschrift:

Ein Rundbodenröhrchen aus Glas (Durchmesser 16 mm, Länge 125 mm) mit Gewinde wurde manuell mit einem Rührer versehen und auf der Capper-Station mit einem Schraubdeckel mit Septum verschlossen. Das Röhrchen wurde von Roboter 1 in den auf 15°C temperierten Reaktorblock gestellt. Roboter 2 pipettierte nacheinander folgende Reagenzien hinzu:
1.) 1 ml einer 0,1 M Amidin-Lösung + 20% HClO₄ in Dichlormethan
2.) 0,5 ml einer 0,3 M Aldehyd-Lösung in Dichlormethan
3.) 0,575 ml einer 0,2 M Isonitril-Lösung in Dichlormethan

Das Reaktionsgemisch wurde bei 15°C in einem der Rührblöcke 660 min lang gerührt. Danach wurde die Reaktionslösung an der Filtrations-Station abfiltriert. Das Röhrchen wurde dabei zweimal mit je 1 ml Dichlormethan und 200 µl Wasser gespült.

Das Rack mit den Röhrchen würde anschließend manuell auf die Aufarbeitungsanlage gestellt. Dort wurde das Reaktionsgemisch auf einem Vortexer mit 3 ml einer 10%igen NaCl-Lösung und 1,5 ml Dichlormethan versetzt. Im Spin-Reaktor wurde zehn Minuten lang gründlich gemischt und durch die langsame Abnahme der Drehbewegung eine deutliche Phasengrenze ausgebildet. Diese Phasengrenze wurde optisch detektiert und die organische Phase abpipettiert. Im nächsten Schritt wurde das Reaktionsgemisch erneut mit 1,5 ml Dichlormethan versetzt. Die Lösung wurde geschüttelt, zentrifugiert und die organische Phase abpipettiert. Die vereinigten organischen Phasen wurden über 2,4 g MgSO₄ (granuliert) getrocknet. Das Lösungsmittel wurde in einer Vakuumzentrifuge entfernt.

Die eingesetzten Chemikalien und Lösungsmittel wurden kommerziell erworben. Jede Substanz wurde mit ESI-MS und/oder NMR analysiert.

### Beispiel 1

### 7-Chlor-2-furan-2-yl-(6-isocyano-hexyl)-imidazo[1,2-a]pyrimidin-3,5-diamin (1)

Verbindung **1** wurde gemäß der allgemeinen Synthesevorschrift aus 1.0 ml 2,6-Diamino-4-chlorpyrimidin-Lösung (0.1 M, DCM), 0.575 ml 1,6-Diisocyanhexan-Lösung (0.2 M, DCM), 0.500 ml Furfural-Lösung (0.3 M, DCM) und 10 µl Perchlorsäure (w= 20%) dargestellt.
Berechnete Masse 360,85; gefundene Masse 359,2 (ESI-MS)

### Beispiel 2

### (5,7-Dimethyl-2-pyridin-2-yl-imidazo[1,2-a]pyrimidin-3-yl)-(6-isocyano-hexyl)-amin (2)

Verbindung **2** wurde gemäß der allgemeinen Synthesevorschrift aus 1.0 ml 2-Amino-4,6-dimethylpyrimidin-Lösung (0.1 M, DCM), 0.575 ml 1,6-Diisocyanhexan-Lösung (0.2 M, DCM), 0.500 ml Pyridin-2-carbaldehyd-Lösung (0.3 M, DCM) und 10 µl Perchlorsäure (w= 20%) dargestellt.
Berechnete Masse 360,85; gefundene Masse 359,2 (ESI-MS)

### Beispiel 3

### (2-Cyclohexyl-5-methyl-imidazo[1,2-a]pyridin-3-yl)-(1,1,3,3-tetramethyl-butyl)-amin (3)

Verbindung **3** wurde gemäß der allgemeinen Synthesevorschrift aus 1.0 ml 2-Amino-6-methylpyridin-Lösung (0.1 M, DCM), 0.575 ml 1,1,3,3-Tetramethylbutylisocyanid-Lösung (0.2 M, DCM), 0.500 ml Cyclohexylcarbaldehyd-Lösung (0.3 M, DCM) und 10 µl Perchlorsäure (w= 20%) dargestellt.
Berechnete Masse 341,54; gefundene Masse 342,4 (ESI-MS)

### Beispiel 4

### 7-Chlor-(1,1,3,3-tetramethylbutyl)-2-thiophen-2-ylimidazo[1,2-a]pyrimidin-3,5-diamin (4)

Verbindung **4** wurde gemäß der allgemeinen Synthesevorschrift aus 1.0 ml 2,6-Diamino-4-chlorpyrimidin-Lösung (0.1 M, DCM), 0.575 ml 1,1,3,3-Tetramethylbutylisocyanid-Lösung (0.2 M, DCM), 0.500 ml Thiophen-2-carbaldehyd-Lösung (0.3 M, DCM) und 10 µl Perchlorsäure (w= 20%) dargestellt.
Berechnete Masse 377,94; gefundene Masse 378,3 (ESI-MS)

### Beispiel 5

### [6-Brom-2-(2-methoxyphenyl)-imidazo[1,2-a]pyridin-3-yl]-(1,1,3,3-tetramethylbutyl)-amin (5)

Verbindung **5** wurde gemäß der allgemeinen Synthesevorschrift aus 1.0 ml 2-Amino-5-brompyridin-Lösung (0.1 M, DCM), 0.575 ml 1,1,3,3-Tetramethylbutylisocyanid-Lösung (0.2 M, DCM), 0.500 ml 2-Methoxy-benzaldehyd-Lösung (0.3 M, DCM) und 10 µl Perchlorsäure (w= 20%) dargestellt.
Berechnete Masse 430,39; gefundene Masse M+H₂O = 447,3 (ESI-MS)

### Beispiel 6

### [6,8-Dibrom-2-(2,3-dimethoxyphenyl)-5-methylimidazo[1,2-a]pyridin-3-yl]-(1,1,3,3-tetramethylbutyl)-amin (6)

Verbindung **6** wurde gemäß der allgemeinen Synthesevorschrift aus 1.0 ml 2-Amino-3,5-dibrom-6-methylpyridin-Lösung (0.1 M, DCM), 0.575 ml 1,1,3,3-Tetramethylbutylisocyanld-Lösung (0.2 M, DCM), 0.500 ml 2,3-Dimethoxybenzaldehyd-Lösung (0.3 M, DCM) und 10 µl Perchlorsäure (w= 20%) dargestellt.
Berechnete Masse 553,34; gefundene Masse M+H₂O = 572,1 (ESI-MS)

### Beispiel 7

### N-[4-(8-Benzyloxy-3-cyclohexylamino-imidazo[1,2-a]pyridin-2-yl)-phenyl]-acetamid (7)

Verbindung **7** wurde gemäß der allgemeinen Synthesevorschrift aus 1.0 ml 2-Amino-3-benzyloxypyridin-Lösung (0.1 M, DCM), 0.575 ml Cyclohexylisocyanid-Lösung (0.2 M, DCM), 0.500 ml 4-Acetamidobenzaldehyd-Lösung (0.3 M, DCM) und 10 µl Perchlorsäure (w= 20%) dargestellt. berechnete Masse 454,58; gefundene Masse 455,4 (ESI-MS)

### Beispiel 8

### 3-(8-Benzyloxy-3-butylamino-imidazo[1,2-a]pyridin-2-yl)-phenol (8)

Verbindung **8** wurde gemäß der allgemeinen Synthesevorschrift aus 1.0 ml 2-Amino-3-benzyloxypyridin-Lösung (0.1 M, DCM), 0.575 ml n-Butylisocyanid-Lösung (0.2 M, DCM), 0.500 ml 3-Hydroxybenzaldehyd-Lösung (0.3 M, DCM) und 10 µl Perchlorsäure (w= 20%) dargestellt.
Berechnete Masse 387,49; gefundene Masse 388,4 (ESI-MS)

### Beispiel 9

### [8-Benzyloxy-2-(3,5-dimethoxyphenyl)-imidazo[1,2-a]pyridin-3-ylamino]-essigsäuremethylester (9)

Verbindung **9** wurde gemäß der allgemeinen Synthesevorschrift aus 1.0 ml 2-Amino-3-benzyloxypyridin-Lösung (0.1 M, DCM), 0.575 ml Isocyanoessigsäuremethylester-Lösung (0.2 M, DCM), 0.500 ml 3,5-Dimethoxybenzaldehyd-Lösung (0.3 M, DCM) und 10 µl Perchlorsäure (w= 20%) dargestellt.
Berechnete Masse 447,50; gefundene Masse 448,3 (ESI-MS)

### Beispiel 10

### [8-Benzyloxy-2-(3,5-dimethoxyphenyl)-imidazo(1,2-a]pyridin-3-yl]-cyclohexyl-amin (10)

Verbindung **10** wurde gemäß der allgemeinen Synthesevorschrift aus 1.0 ml 2-Amino-3-benzyloxypyridin-Lösung (0.1 M, DCM), 0.575 ml Cyclohexylisocyanid-Lösung (0.2 M, DCM), 0.500 ml 3,5-Dimethoxy-benzaldehyd-Lösung (0.3 M, DCM) und 10 µl Perchlorsäure (w= 20%) dargestellt.
Berechnete Masse 457,58; gefundene Masse 458,5 (ESI-MS)

### Beispiel 11

### Cyclohexyl-[6,8-dibrom-2-(2-methoxyphenyl)-5-methylimidazo[1,2-a]pyridin-3-yl]-amin (11)

Verbindung **11** wurde gemäß der allgemeinen Synthesevorschrift aus 1.0 ml 2-Amino-3,5-dibrom-6-methylpyridin-Lösung (0.1 M, DCM), 0.575 ml Cyclohexylisocyanid-Lösung (0.2 M, DCM), 0.500 ml 2-Methoxybenzaldehyd-Lösung (0.3 M, DCM) und 10 µl Perchlorsäure (w= 20%) dargestellt.
Berechnete Masse 457,58; gefundene Masse 458,5 (ESI-MS)

### Beispiel 12

### 3-[3-(2,6-Dimethylphenylamino)-6-nitro-imidazo[1,2-a]pyridin-2-yl]-phenol (12)

Verbindung **12** wurde gemäß der allgemeinen Synthesevorschrift aus 1.0 ml 2-Amino-5-nitropyridin-Lösung (0.1 M, DCM), 0.575 ml 2,6-Dimethylphenylisocyanid-Lösung (0.2 M, DCM), 0.500 ml 3-Hydroxybenzaldehyd-Lösung (0.3 M, DCM) und 10 µl Perchlorsäure (w= 20%) dargestellt.
Berechnete Masse 376,42; gefundene Masse 375,3 (ESI-MS)

### Beispiel 13

### [6-Brom-2-(2-methoxyphenyl)-imidazo[1,2-a]pyridin-3-yl]-(1,1,3,3-tetramethylbutyl)-amin (13)

Verbindung **13** wurde gemäß der allgemeinen Synthesevorschrift aus 1.0 ml 2-Amino-5-brompyridin-Lösung (0.1 M, DCM), 0.575 ml 1,1,3,3-Tetramethylbutylisocyanid-Lösung (0.2 M, DCM), 0.500 ml 2-Methoxybenzaldehyd-Lösung (0.3 M, DCM) und 10 µl Perchlorsäure (w= 20%) dargestellt.
Berechnete Masse 430,39; gefundene Masse M+H₂O = 447,3 (ESI-MS)

### Beispiel 14

### [6,8-Dibrom-2-(2,3-dimethoxyphenyl)-5-methylimidazo[1,2-a]pyridin-3-yl]-(1,1,3,3-tetramethylbutyl)-amin (14)

Verbindung **14** wurde gemäß der allgemeinen Synthesevorschrift aus 1.0 ml 2-Amino-3,5-dibrom-6-methylpyridin-Lösung (0.1 M, DCM), 0.575 ml 1,1,3,3-Tetramethylbutylisocyanid-Lösung (0.2 M, DCM), 0.500 ml 2,3-Dimethoxy-benzaldehyd-Lösung (0.3 M, DCM) und 10 µl Perchlorsäure (w= 20%) dargestellt.
Berechnete Masse 553,34; gefundene Masse M+H₂O = 572,1 (ESI-MS)

### Beispiel 15

### Cyclohexyl-(2-phenyl-imidazo[1,2-a]chinolin-1-yl)-amin (15)

Verbindung **15** wurde gemäß der allgemeinen Synthesevorschrift aus 1.0 ml 2-Aminochinolin-Lösung (0.1 M, DCM), 0.575 ml Cyclohexylisocyanid-Lösung (0.2 M, DCM), 0.500 ml Benzaldehyd-Lösung (0.3 M, DCM) und 10 µl Perchlorsäure (w= 20%) dargestellt.
Berechnete Masse 341,46; gefundene Masse 342,3 (ESI-MS)

### Beispiel 16

### [6-(2-Cyclohexyl-5-methyl-imidazo[1,2-a]pyridin-3-ylamino)-hexyl]-methylidyn-ammonium (16)

Verbindung **16** wurde gemäß der allgemeinen Synthesevorschrift aus 1.0 ml 2-Amino-6-methylpyridin-Lösung (0.1 M, DCM), 0.575 ml 1,6-Diisocyanhexan-Lösung (0.2 M, DCM), 0.500 ml Cyclohexylcarbaldehyd-Lösung (0.3 M, DCM) und 10 µl Perchlorsäure (w= 20%) dargestellt.
Berechnete Masse 339,5; gefundene Masse = 339,4(ESI-MS)

### Beispiel 17

### (2,6-Dimethyl-imidazo[1,2-a]pyridin-3-yl)-(1,1,3,3-tetramethylbutyl)-amin (17)

Verbindung **17** wurde gemäß der allgemeinen Synthesevorschrift aus 1.0 ml 2-Amino-5-methylpyridin-Lösung (0.1 M, DCM), 0.575 ml 1,1,3,3-Tetramethylbutylisocyanid -Lösung (0.2 M, DCM), 0.500 ml Acetaldehyd-Lösung (0.3 M, DCM) und 10 µl Perchlorsäure (w= 20%) dargestellt.
Berechnete Masse 273,4; gefundene Masse = 274,3(ESI-MS)

### Beispiel 18

### Cyclohexyl-(2,7-dimethyl-imidazo[1,2-a]pyridin-3-yl)-amin (18)

Verbindung **18** wurde gemäß der allgemeinen Synthesevorschrift aus 1.0 ml 2-Amino-4-methylpyridin-Lösung (0.1 M, DCM), 0.575 ml Cyclohexylisocyanid-Lösung (0.2 M, DCM), 0.500 ml Acetaldehyd-Lösung (0.3 M, DCM) und 10 µl Perchlorsäure (w= 20%) dargestellt. Berechnete Masse 243,4; gefundene Masse = 244,4 (ESI-MS)

### Beispiel 19

### Cyclohexyl-(2,5,7-trimethyl-imidazo[1,2-a]pyridin-3-yl)-amin (19)

Verbindung **19** wurde gemäß der allgemeinen Synthesevorschrift aus 1.0 ml 2-Amino-4,6-dimethylpyridin-Lösung (0.1 M, DCM), 0.575 ml Cyclohexylisocyanid-Lösung (0.2 M, DCM), 0.500 ml Acetaldehyd-Lösung (0.3 M, DCM) und 10 µl Perchlorsäure (w= 20%) dargestellt.
Berechnete Masse 257,4; gefundene Masse = 258,4 (ESI-MS)

### Beispiel 20

### [2-(3,4-Dimethoxyphenyl)-6-methyl-imidazo[1,2-a]pyridin-3-yl]-(6-isocyanohexyl)-amin (20)

Verbindung **20** wurde gemäß der allgemeinen Synthesevorschrift aus 1.0 ml 2-Amino-5-methylpyridin-Lösung (0.1 M, DCM), 0.575 ml 1,6-Diisocyanhexan-Lösung (0.2 M, DCM), 0.500 ml 3,4-Dimethoxybenzaldehyd-Lösung (0.3 M, DCM) und 10 µl Perchlorsäure (w= 20%) dargestellt.
Berechnete Masse 394,5; gefundene Masse = 393,4(ESI-MS)

### Beispiel 21

### (2,7-Dimethyl-imidazo[1,2-a]pyridin-3-yl)-(1,1,3,3-tetramethyl-butyl)-amin (21)

Verbindung **21** wurde gemäß der allgemeinen Synthesevorschrift aus 1.0 ml 2-Amino-4-methylpyridin-Lösung (0.1 M, DCM), 0.575 ml 1,1,3,3-Tetramethylbutyl-Lösung (0.2 M, DCM), 0.500 ml Acetaldehyd-Lösung (0.3 M, DCM) und 10 µl Perchlorsäure (w= 20%) dargestellt.
Berechnete Masse 273,4; gefundene Masse = 274,3(ESI-MS)

### Beispiel 22

### (2,8-Dimethyl-imidazo[1,2-a]pyridin-3-yl)-(1,1,3,3-tetramethyl-butyl)-amin (22)

Verbindung **22** wurde gemäß der allgemeinen Synthesevorschrift aus 1.0 ml 2-Amino-3-methylpyridin-Lösung (0.1 M, DCM), 0.575 ml 1,1,3,3-Tetramethylbutyl-Lösung (0.2 M, DCM), 0.500 ml Acetaldehyd-Lösung (0.3 M, DCM) und 10 µl Perchlorsäure (w= 20%) dargestellt.
Berechnete Masse 273,4; gefundene Masse = 274,4 (ESI-MS)

### Beispiel 23

### (1,1,3,3-Tetramethyl-butyl) - (2,5,7-trimethylimidazo[1,2-a]pyridin-3-yl)-amin (23)

Verbindung **23** wurde gemäß der allgemeinen Synthesevorschrift aus 1.0 ml 2-Amino-4,6-dimethylpyridin-Lösung (0.1 M, DCM), 0.575 ml 1,1,3,3-Tetramethylbutyl-Lösung (0.2 M, DCM), 0.500 ml Acetaldehyd-Lösung (0.3 M, DCM) und 10 µl Perchlorsäure (w= 20%) dargestellt.
Berechnete Masse 287,4; gefundene Masse = 288,4(ESI-MS)

### Beispiel 24

### [2-(3,4-Dimethoxy-phenyl)-7-methyl-imidazo[1,2-a]pyridin-3-yl]-(6-isocyano-hexyl)-amin (24)

Verbindung **24** wurde gemäß der allgemeinen Synthesevorschrift aus 1.0 ml 2-Amino-4-methylpyridin-Lösung (0.1 M, DCM), 0.575 ml 1,6-Diisocyanhexan-Lösung (0.2 M, DCM), 0.500 ml 3,4-Dimethoxybenzaldehyd-Lösung (0.3 M, DCM) und 10 µl Perchlorsäure (w= 20%) dargestellt.
Berechnete Masse 394, 5; gefundene Masse = 393,4(ESI-MS)

### Beispiel 25

### (6-Isocyano-hexyl)-[2-(2-methoxy-phenyl)-6-methylimidazo[1,2-a]pyridin-3-yl]-amin (25)

Verbindung **25** wurde gemäß der allgemeinen Synthesevorschrift aus 1.0 ml 2-Amino-5-methylpyridin-Lösung (0.1 M, DCM), 0.575 ml 1,6-Diisocyanhexan-Lösung (0.2 M, DCM), 0.500 ml 3,4-Dimethoxybenzaldehyd-Lösung (0.3 M, DCM) und 10 µl Perchlorsäure (w= 20%) dargestellt.
Berechnete Masse 363,5; gefundene Masse = 363,4(ESI-MS)

### Beispiel 26

### Cyclohexyl-(2-furan-2-yl-6-trifluoromethyl-imidazo[1,2-a]pyridin-3-yl)-amin (26)

Verbindung **26** wurde gemäß der allgemeinen Synthesevorschrift aus 1.0 ml 2-Amino-4-trifluormethylpyridin-Lösung (0.1 M, DCM), 0.575 ml Cyclohexylisocyanid -Lösung (0.2 M, DCM), 0.500 ml Furfural-Lösung (0.3 M, DCM) und 10 µl Perchlorsäure (w= 20%) dargestellt.
Berechnete Masse 349,3; gefundene Masse = 350,3(ESI-MS)

### Beispiel 27

### (8-Benzyloxy-2-cyclohexyl-imidazo[1,2-a]pyridin-3-yl)-cyclohexyl-amin (27)

Verbindung **27** wurde gemäß der allgemeinen Synthesevorschrift aus 1.0 ml 2-Amino-3-benzyloxypyridin-Lösung (0.1 M, DCM), 0.575 ml Cyclohexylisocyanid -Lösung (0.2 M, DCM), 0.500 ml Cyclohexylcarbaldehyd-Lösung (0.3 M, DCM) und 10 µl Perchlorsäure (w= 20%) dargestellt.
Berechnete Masse 403,6; gefundene Masse = 404,4 (ESI-MS)

### Beispiel 28

### (8-Benzyloxy-2-methyl-imidazo[1,2-a]pyridin-3-yl)-cyclohexyl-amin (28)

Verbindung **28** wurde gemäß der allgemeinen Synthesevorschrift aus 1.0 ml 2-Amino-3-benzyloxypyridin-Lösung (0.1 M, DCM), 0.575 ml Cyclohexylisocyanid -Lösung (0.2 M, DCM), 0.500 ml Acetaldehyd-Lösung (0.3 M, DCM) und 10 µl Perchlorsäure (w= 20%) dargestellt.
Berechnete Masse 335,4; gefundene Masse = 336,3 (ESI-MS)

### Beispiel 29

### (8-Benzyloxy-2-methyl-imidazo[1,2-a]pyridin-3-yl)-(1,1,3,3-tetramethyl-butyl)-amin (29)

Verbindung **29** wurde gemäß der allgemeinen Synthesevorschrift aus 1.0 ml 2-Amino-3-benzyloxypyridin-Lösung (0.1 M, DCM), 0.575 ml 1,1,3,3-Tetramethylbutylisocyanid-Lösung (0.2 M, DCM), 0.500 ml Acetaldehyd-Lösung (0.3 M, DCM) und 10 µl Perchlorsäure (w= 20%) dargestellt.
Berechnete Masse 365,5; gefundene Masse = 366,5 (ESI-MS)

### Beispiel 30

### (8-Benzyloxy-2-cyclohexyl-imidazo[1,2-a]pyridin-3-ylamino)-essigsäuremethylester (30)

Verbindung **30** wurde gemäß der allgemeinen Synthesevorschrift aus 1.0 ml 2-Amino-3-benzyloxypyridin-Lösung (0.1 M, DCM), 0.575 ml Isocyanoessigsäuremethylester -Lösung (0.2 M, DCM), 0.500 ml Cyclohexylcarbaldehyd-Lösung (0.3 M, DCM) und 10 µl Perchlorsäure (w= 20%) dargestellt.
Berechnete Masse 393,5; gefundene Masse = 394,5 (ESI-MS)

### Beispiel 31

### (8-Benzyloxy-2-methyl-imidazo[1,2-a]pyridin-3-ylamino)-essigsäuremethylester (31)

Verbindung **31** wurde gemäß der allgemeinen Synthesevorschrift aus 1.0 ml 2-Amino-3-benzyloxypyridin-Lösung (0.1 M, DCM), 0.575 ml Isocyanoessigsäuremethylester -Lösung (0.2 M, DCM), 0.500 ml Acetaldehyd-Lösung (0.3 M, DCM) und 10 µl Perchlorsäure (w= 20%) dargestellt.
Berechnete Masse 325,4; gefundene Masse = 326,3 (ESI-MS)

### Beispiel 32

### Butyl-(2-cyclohexyl-5-propyl-imidazo[1,2-a]pyridin-3-yl)-amin (32)

Verbindung **32** wurde gemäß der allgemeinen Synthesevorschrift aus 1.0 ml 2-Amino-6-propylpyridin-Lösung (0.1 M, DCM), 0.575 ml n-Butylisocyanid-Lösung (0.2 M, DCM), 0.500 ml Cyclohexylcarbaldehyd-Lösung (0.3 M, DCM) und 10 µl Perchlorsäure (w= 20%) dargestellt.
Berechnete Masse 313,5; gefundene Masse = 314,5 (ESI-MS)

### Beispiel 33

### N-Cyclohexyl-N-(6,8-dichlor-2-furan-2-yl-imidazo[1,2-a]pyridin-3-yl)-acetamid (33)

Verbindung **33** wurde aus 1.0 ml 2-Amino-3,5-dichlorpyridin-Lösung (0.1 M, DCM), 0.575 ml Cyclohexylisocyanid-Lösung (0.2 M, DCM), 0.500 ml Furfural-Lösung (0.3 M, DCM) und 10 µl Perchlorsäure (w= 20%) und Umsetzung mit Acetylchlorid dargestellt, wobei das überschüssige Acetylchlorid im Vakuum entfernt wurde.
Berechnete Masse 392,3; gefundene Masse = 392,3/394,3; M-Acetyl 350,4 (ESI-MS)

### Beispiel 34

### N-Cyclohexyl-N-(2-furan-2-yl-6-trifluormethylimidazo[1,2-a]pyridin-3-yl)-acetamid (34)

Verbindung **34** wurde aus 1.0 ml 2-Amino-5-trifluormethylpyridin-Lösung (0.1 M, DCM), 0.575 ml Cyclohexylisocyanid-Lösung (0.2 M, DCM), 0.500 ml Furfural-Lösung (0.3 M, DCM) und 10 µl Perchlorsäure (w= 20%) und Umsetzung mit Acetylchlorid dargestellt, wobei das überschüssige Acetylchlorid im Vakuum entfernt wurde.
Berechnete Masse 391,4; gefundene Masse = 392,3; M-Acetyl 350,4 (ESI-MS)

### Beispiel 35

### N-(8-Benzyloxy-2-cyclohexyl-imidazo[1,2-a]pyridin-3-yl)-N-cyclohexyl-acetamid (35)

Verbindung **35** wurde aus 1.0 ml 2-Amino-3-benzyloxypyridin-Lösung (0.1 M, DCM), 0.575 ml Cyclohexylisocyanid-Lösung (0.2 M, DCM), 0.500 ml Cyclohexylcarbaldehyd-Lösung (0.3 M, DCM) und 10 µl Perchlorsäure (w= 20%) und Umsetzung mit Acetylchlorid dargestellt, wobei das überschüssige Acetylchlorid im Vakuum entfernt wurde.
Berechnete Masse 445,6; gefundene Masse = 446,4; M-Acetyl 404,4 (ESI-MS)

### Beispiel 36

### (5-Methyl-2-phenanthren-9-yl-imidazo[1,2-a]pyridin-3-yl)-(1,1,3,3-tetramethyl-butyl)-amin (36)

Verbindung **36** wurde gemäß der allgemeinen Synthesevorschrift aus 1.0 ml 2-Amino-6-methylpyridin-Lösung (0.1 M, DCM) , 0.575 ml 1,1,3,3-Tetramethylbutylisocyanid-Lösung (0.2 M, DCM), 0.500 ml Phenanthren-9-carbaldehyd-Lösung (0.3 M, DCM) und 10 µl Perchlorsäure (w= 20%) dargestellt.
Berechnete Masse 435,6; gefundene Masse = 436,5 (ESI-MS)

### Beispiel 37

### (2-Anthracen-9-yl-7-methyl-imidazo[1,2-a]pyrimidin-3-yl)-(1,1,3,3-tetramethyl-butyl)-amin (37)

Verbindung **37** wurde gemäß der allgemeinen Synthesevorschrift aus 1.0 ml 2-Amino-4-methylpyrimidin-Lösung (0.1 M, DCM), 0.575 ml 1,1,3,3-Tetramethylbutylisocyanid-Lösung (0.2 M, DCM), 0.500 ml Anthracen-9-carbaldehyd-Lösung (0.3 M, DCM) und 10 µl Perchlorsäure (w= 20%) dargestellt.
Berechnete Masse 436,6; gefundene Masse = 437,3 (ESI-MS)

### Beispiel 38

### Cyclohexyl-[7-methyl-2-(1-methyl-1H-pyrrol-2-yl)-imidazo[1,2-a]pyrimidin-3-yl]-amin (38)

Verbindung **38** wurde gemäß der allgemeinen Synthesevorschrift aus 1.0 ml 2-Amino-4-methylpyrimidin-Lösung (0.1 M, DCM), 0.575 ml Cyclohexylisocyanid-Lösung (0.2 M, DCM), 0.500 ml N-Methylpyrrol-2-carbaldehyd-Lösung (0.3 M, DCM) und 10 µl Perchlorsäure (w= 20%) dargestellt.
Berechnete Masse 309,4; gefundene Masse = 310,4 (ESI-MS)

Die erfindungsgemäßen Verbindungen erwiesen sich als Liganden des schmerzrelevanten α2-Subtyps des humanen α-adrenergen Rezeptors. Die Bestimmung der Affinität zum α2-Subtyps des humanen α-adrenergen Rezeptors erfolgte mittels eines für das High Throughput Screening üblichen SPA-Assays, wie er in John P. Devlin, High Throughput Screening, Marcel Dekker Inc. 1997, Seite 307 bis 316, beschrieben ist. Diese Literatur wird hiermit als Referenz eingeführt und gilt somit als Teil der Offenbarung. Bei einer Konzentration von 10 µM wurden beispielhaft die folgenden Affinitäten bestimmt:

| | alpha2-Affinität, 10µM |
|---|---|
| Beispiel 16 | 67 % |
| Beispiel 17 | 65 % |
| Beispiel 18 | 74 % |
| Beispiel 19 | 81 % |
| Beispiel 20 | 85 % |
| Beispiel 21 | 109 % |
| Beispiel 22 | 69 % |
| Beispiel 23 | 97 % |
| Beispiel 24 | 75 % |
| Beispiel 25 | 51 % |
| Beispiel 26 | 70 % |
| Beispiel 27 | 77 % |
| Beispiel 28 | 80 % |
| Beispiel 29 | 100 % |
| Beispiel 30 | 60 % |
| Beispiel 31 | 61 % |
| Beispiel 32 | 61 % |
| Beispiel 33 | 73 % |
| Beispiel 34 | 72 % |
| Beispiel 35 | 75 % |

## Patentansprüche

1. Bicyclische Imidazo-3-yl-amine der allgemeinen Formel I, 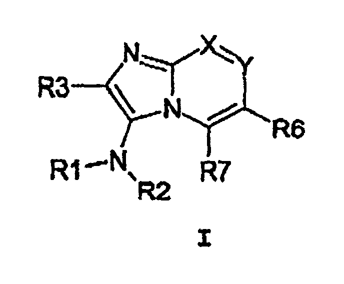 worin
X CR⁴ oder N und Y CR⁵ oder N bedeuten mit der Maßgabe, daß X und Y nicht gleichzeitig N bedeuten,
R¹ (CH₂)ₙCN mit n = 4 ,5 oder 6, gegebenenfalls substituiertes Phenyl, C₄-C₈-Cycloalkyl, CH₂CH₂R (R = 4-Morpholino), 1,1,3,3,-Tetramethylbutyl oder CH₂R^{a}, wobei R^{a} für Wasserstoff, OH, C₁-C₈-Alkyl (verzweigt oder unverzweigt), gegebenenfalls substituiertes Phenyl, CO(OR') (mit R' = unverzweigtes C₁-C₄-Alkyl oder verzweigtes C₁-C₅-Alkyl), PO(OR')₂ (mit R' = unverzweigtes C₁-C₄-Alkyl oder verzweigtes C₁-C₅-Alkyl) oder Si(R^{x}R^{y}R^{z}) (mit R^{x}, R^{y}, und R^{z} jeweils unabhängig voneinander C₁-C₄-Alkyl(verzweigt oder unverzweigt), C₄-C₈-Cycloalkyl oder Phenyl) steht, bedeutet,
R² Wasserstoff, COR^{b}, wobei R^{b} für C₁-C₄-Alkyl (verzweigt oder unverzweigt) oder C₃-C₈-Cycloalkyl steht, CH₂CH₂CO(OR^{c}), wobei R^{c} für C₁-C₄-Alkyl (verzweigt oder unverzweigt), Adamantyl, gegebenenfalls substituiertes Phenyl, gegebenenfalls substituiertes 1-Naphtyl oder 2-Naphtyl oder jeweils gegebenenfalls substiuiertes 2-Pyridyl, 3-Pyridyl, 4-Pyridyl, Thiazolyl oder Furoyl steht, CH₂Phenyl, CH₂CH₂R^{d}, wobei R^{d} für gegebenenfalls substituiertes Phenyl steht, oder CONHR^{e}, wobei R^{e} für C₁-C₈-Alkyl(verzweigt oder unverzweigt), C₃-C₈-Cycloalkyl oder gegebenenfalls substiuiertes Phenyl steht, bedeutet
R³ C₁-C₈-Alkyl(verzweigt oder unverzweigt), C₃-C₈-Cycloalkyl, gegebenenfalls substituiertes Phenyl, gegebenenfalls substituiertes Naphtyl, gegebenenfalls substituiertes Pyrrol, gegebenenfalls substituiertes Pyridyl, gegebenenfalls substituiertes Furan, gegebenenfalls substituiertes Thiophen, gegebenenfalls substituiertes Anthracen, gegebenenfalls substituiertes Phenanthren oder gegebenenfalls substituiertes Chinolin bedeutet,
R⁴, R⁵, R⁶ und R⁷ ausgewählt sind aus der Gruppe Wasserstoff, NO₂, NH₂, OH, CF₃, Cl, F, Br, CN, Methyl oder OR'' mit R'' = Benzyl oder R⁶ und R⁷ gemeinsam eine Brücke -CH=CH-CH=CH- bilden und die Reste R⁴ und R⁵, soweit vorhanden, Wasserstoff bedeuten.
mit der Maßgabe, daß mindestens einer der im Molekül vorhandenen Reste R⁴, R⁵, R⁶ oder R⁷ nicht Wasserstoff ist und, wenn R¹ Methyl, Ethyl , Propyl, n-Butyl, iso-Butyl, CO(OMethyl) oder Benzyl bedeutet, R³ nicht Methyl bedeutet, und, wenn einer der Reste R⁴, R⁵, R⁶ und R⁷ OBenzyl bedeutet oder R⁴ OC₁-C₄-Alkyl (verzweigt oder unverzweigt) bedeutet, R¹ nicht CH₂R^{a} (wobei R^{a} für Wasserstoff oder C₁-C₅-Alkyl (verzweigt oder unverzweigt)steht) bedeutet , in Form der Basen oder von pharmazeutisch akzeptablen Salzen.

2. Bicyclische Imidazo-3-yl-amine nach Anspruch 1, **dadurch gekennzeichnet, daß** R² Wasserstoff bedeutet,
R¹ ausgewählt ist aus der Gruppe (CH₂)ₙCN mit n = 4, 5 oder 6, Cyclohexyl, CH₂CO(OMethyl), 2,6-Dimethylphenyl, 1,1,3,3,-Tetramethylbutyl oder n-Butyl und
R³ ausgewählt ist aus der Gruppe 2-Pyridyl, 3-Pyridyl, 2-Furanyl, 2-Pyrroyl, Methyl, tert-Butyl, 3-Hydroxyphenyl, 3,4-Dimethoxyphenyl, 2,3-Dichlorphenyl, 2,4-Dichlorphenyl, 2-Methoxyphenyl, 2,3-Dimethoxyphenyl, 3-Bromphenyl, 4-Brom-2-fluorphenyl, 5-Brom-2-fluorphenyl, 3-Brom-4-fluorphenyl, 3-Chlorphenyl, 3,4-Dichlorphenyl, 3-Fluorphenyl , 3-Methylphenyl, 3 -Phenoxyphenyl, 3-(4-Chlorphenoxy)phenyl, 2-Chlor-4-fluorphenyl, 2-Chlor-6-fluorphenyl, 2,4-Dimethylphenyl, 2,5-Dimethylphenyl, 2-Bromphenyl, 2-Fluorphenyl, 2-(Trifluormethyl)-phenyl.

3. Bicyclische Imidazo-3-yl-amine gemäß einem der Ansprüche 1 bis 2 **dadurch gekennzeichnet, daß** es sich um
7-Chlor-2-furan-2-yl-(6-isocyanohexyl)-imidazo[1,2-a]pyrimidin-3,5-diamin,
(5,7-Dimethyl-2-pyridin-2-yl-imidazo[1, 2-a]pyrimidin-3-yl)-(6-isocyanohexyl)-amin,
7-Chlor-(1,1,3,3-tetramethylbutyl)-2-thiophen-2-ylimidazo [1,2-a]pyrimidin-3,5-diamin,
[6-Brom-2-(2-methoxyphenyl)-imidazo[1,2-a]pyridin-3-yl]-(1,1,3,3-tetramethylbutyl)-amin,
*N*-[4-(8-Benzyloxy-3-cyclohexylamino-imidazo[1,2-a]pyridin-2-yl)-phenyl]-acetamid,
3-(8-Benzyloxy-3-butylamino-imidazo[1,2-a]pyridin-2-yl)-phenol,
[8-Benzyloxy-2-(3,5-dimethoxyphenyl)-imidazo[1,2-a]pyridin-3-ylamino]-essigsäuremethylester,
[8-Benzyloxy-2-(3,5-dimethoxyphenyl)-imidazo[1,2-a] pyridin-3-yl]-cyclohexyl-amin,
Cyclohexyl-[6,8-dibrom-2-(2-methoxyphenyl)-5-methyl-imidazo[1,2-a]pyridin-3-yl]-amin,
3-[3-(2,6-Dimethylphenylamino)-6-nitro-imidazo[1,2-a]pyridin-2-yl]-phenol,
[6-Brom-2-(2-methoxyphenyl)-imidazo[1,2-a]pyridin-3-yl]-(1,1,3,3-tetramethylbutyl)-amin,
[6,8-Dibrom-2-(2,3-dimethoxyphenyl)-5-methylimidazo[1,2-a]pyridin-3-yl]-(1,1,3,3-tetramethylbutyl)-amin,
Cyclohexyl-(2-phenyl-imidazo[1,2-a]chinolin-1-yl)-amin,
[6-(2-Cyclohexyl-5-methyl-imidazo[1,2-a]pyridin-3-ylamino)-hexyl]-methylidyn-ammonium,
(2,6-Dimethyl-imidazo[1,2-a]pyridin-3-yl)-(1,1,3,3-tetramethylbutyl)-amin,
Cyclohexyl-(2,7-dimethyl-imidazo[1,2-a]pyridin-3-yl)-amin,
Cyclohexyl-(2,5,7-trimethyl-imidazo[1,2-a]pyridin-3-yl)-amin,
[2-(3,4-Dimethoxyphenyl)-6-methyl-imidazo[1,2-a]pyridin-3-yl]-(6-isocyanohexyl) -amin,
(2,7-Dimethyl-imidazo[1,2-a]pyridin-3-yl)-(1,1,3,3-tetramethyl-butyl)-amin,
(2,8-Dimethyl-imidazo[1,2-a]pyridin-3-yl)-(1,1,3,3-tetramethyl-butyl)-amin
(1,1,3,3-Tetramethyl-butyl)-(2,5,7-trimethylimidazo[1,2-a]pyridin-3-yl)-amin,
[2-(3,4-Dimethoxy-phenyl)-7-methyl-imidazo[1,2-a]pyridin-3-yl]-(6-isocyano-hexyl)-amin,
(6-Isocyano-hexyl)-[2-(2-methoxy-phenyl)-6-methylimidazo[1,2-a]pyridin-3-yl]-amin,
Cyclohexyl-(2-furan-2-yl-6-trifluoromethylimidazo[1,2-a]pyridin-3-yl)-amin;
(8-Benzyloxy-2-cyclohexyl-imidazo[1,2-a]pyridin-3-yl)-cyclohexyl-amin,
(8-Benzyloxy-2-methyl-imidazo[1,2-a]pyridin-3-yl)-cyclohexyl-amin,
(8-Benzyloxy-2-methyl-imidazo[1,2-a]pyridin-3-yl)-(1,1,3,3-tetramethyl-butyl)-amin,
(8-Benzyloxy-2-cyclohexyl-imidazo[1,2-a]pyridin-3-ylamino)-essigsäuremethylester,
(8-Benzyloxy-2-methyl-imidazo[1,2-a]pyridin-3-ylamino) - essigsäuremethylester,
Butyl-(2-cyclohexyl-5-propyl-imidazo[1,2-a]pyridin-3-yl)-amin,
N-Cyclohexyl-N-(6,8-dichlor-2-furan-2-ylimidazo[1,2-a]pyridin-3-yl)-acetamid,
N-Cyclohexyl-N-(2-furan-2-yl-6-trifluormethylimidazo[1,2-a]pyridin-3-yl)-acetamid,
N- (8-Benzyloxy-2-cyclohexyl-imidazo[1,2-a]pyridin-3-yl)-N-cyclohexyl-acetamid,
(5-Methyl-2-phenanthren-9-yl-imidazo[1,2,a]pyridin-3-yl)-(1,1,3,3-tetramethyl-butyl)-amin,
(2-Anthracen-9-yl-7-methyl-imidazo[1,2-a]pyrimidin-3-yl)-(1,1,3,3-tetramethyl-butyl)-amin oder Cyclohexyl-[7-methyl-2-(1-methyl-1H-pyrrol-2-yl)-imidazo[1,2-a]pyrimidin-3-yl]-amin handelt.

4. Arzneimittel enthaltend als Wirkstoff mindestens ein bicyclisches Imidazo-3-yl-amin der allgemeinen Formel I gemäß Anspruch 1 , in der R¹ bis R⁷, X und Y die in Anspruch 1 angegebene Bedeutung haben, in Form der freien Base oder eines pharmazeutisch akzeptablen Salzes.

5. Arzneimittel gemäß Anspruch 4, **dadurch gekennzeichnete daß** es als Wirkstoff mindestens ein bicyclisches Imidazo-3-yl-amin ausgewählt aus der Gruppe
7-Chlor-2-furan-2-yl-(6-isocyano-hexyl)-imidazo [1,2-a]pyrimidin-3,5-diamin,
(5,7-Dimethyl-2-pyridin-2-yl-imidazo[1,2-a]pyrimidin-3-yl)-(6-isocyanohexyl)-amin,
7-Chlor-(1,1,3,3-tetramethylbutyl)-2-thiophen-2-yl-imidazo[1,2-a]pyrimidin-3,5-diamin,
[6-Brom-2-(2-methoxyphenyl)-imidazo [1,2-a]pyridin-3-yl]-(1,1,3,3-tetramethylbutyl)-amin,
*N*-[4-(8-Benzyloxy-3-cyclohexylamino-imidazo[1,2-a]pyridin-2-yl)-phenyl]-acetamid,
3-(8-Benzyloxy-3-butylamino-imidazo[1,2 -a] pyridin-2-yl)-phenol,
[8-Benzyloxy-2-(3,5-dimethoxyphenyl)-imidazo[1,2-a]pyridin-3-ylamino]-essigsäuremethylester,
[8-Benzyloxy-2-(3,5-dimethoxyphenyl)-imidazo[1,2-a]pyridin-3-yl]-cyclohexyl-amin,
Cyclohexyl- [6,8-dibrom-2-(2-methoxyphenyl) -5-methyl-imidazo[1,2-a]pyridin-3-yl]-amin,
3-[3-(2,6-Dimethylphenylamino)-6-nitro-imidazo[1,2-a]pyridin-2-yl]-phenol,
[6-Brom-2-(2-methoxyphenyl)-imidazo[1,2-a]pyridin-3-yl]-(1,1,3,3-tetramethylbutyl)-amin,
[6,8-Dibrom-2-(2,3-dimethoxyphenyl)-5-methylimidazo [1,2-a]pyridin-3-yl]-(1,1,3,3-tetramethylbutyl)-amin oder
Cyclohexyl-(2-phenyl-imidazo[1,2-a]chinolin-1-yl)-amin
[6-(2-Cyclohexyl-5-methyl-imidazo[1,2-a]pyridin-3-ylamino)-hexyl]-methylidyn-ammonium,
(2,6-Dimethyl-imidazo[1,2-a]pyridin-3-yl)-(1,1,3,3-tetramethylbutyl)-amin,
Cyclohexyl-(2,7-dimethyl-imidazo[1,2-a]pyridin-3-yl)-amin,
Cyclohexyl-(2,5,7-trimethyl-imidazo[1,2-a]pyridin-3-yl)-amin,
[2-(3,4-Dimethoxyphenyl)-6-methyl-imidazo[1,2-a]pyridin-3-yl]-(6-isocyanohexyl)-amin,
(2,7-Dimethyl-imidazo[1,2-a]pyridin-3-yl)-(1,1,3,3-tetramethyl-butyl)-amin,
(2,8-Dimethyl-imidazo[1,2-a]pyridin-3-yl)-(1,1,3,3-tetramethyl-butyl)-amin
(1,1,3,3-Tetramethyl-butyl)-(2,5,7-trimethylimidazo[1,2-a]pyridin-3-yl)-amin,
[2-(3,4-Dimethoxy-phenyl)-7-methyl-imidazo[1,2-a]pyridin-3-yl]-(6-isocyano-hexyl)-amin,
(6-Isocyano-hexyl)-[2-(2-methoxy-phenyl)-6-methylimidazo [1,2-a]pyridin-3-yl]-amin,
Cyclohexyl-(2-furan-2-yl-6-trifluoromethylimidazo[1,2-a]pyridin-3-yl)-amin;
(8-Benzyloxy-2-cyclohexyl-imidazo[1,2-a]pyridin-3-yl)-cyclohexyl-amin,
(8-Benzyloxy-2-methyl-imidazo[1,2-a]pyridin-3-yl)-cyclohexyl-amin,
(8-Benzyloxy-2-methyl-imidazo[1,2-a]pyridin-3-yl)-(1,1,3,3-tetramethyl-butyl)-amin,
(8-Benzyloxy-2-cyclohexyl-imidazo[1,2-a]pyridin-3-ylamino)-essigsäuremethylester,
(8-Benzyloxy-2-methyl-imidazo[1,2-a]pyridin-3-ylamino)- essigsäuremethylester,
Butyl-(2-cyclohexyl-5-propyl-imidazo[1,2-a]pyridin-3-yl)-amin,
N-Cyclohexyl-N-(6,8-dichlor-2-furan-2-ylimidazo[1,2-a]pyridin-3-yl)-acetamid,
N-Cyclohexyl-N-(2-furan-2-yl-6-trifluormethylimidazo[1,2-a]pyridin-3-yl)-acetamid,
N- (8-Benzyloxy-2-cyclohexyl-imidazo[1,2-a]pyridin-3-yl)-N-cyclohexyl-acetamid,
(5-Methyl-2-phenanthren-9-yl-imidazo[1,2-a]pyridin-3-yl)-(1,1,3,3-tetramethyl-butyl)-amin,
(2-Anthracen-9-yl-7-methyl-imidazo[1,2-a]pyrimidin-3-yl)-(1,1,3,3-tetramethyl-butyl)-amin,
Cyclohexyl-[7-methyl-2-(1-methyl-1H-pyrrol-2-yl)-imidazo[1,2-a]pyrimidin-3-yl]-amin oder der pharmazeutisch akzeptablen Salze dieser Verbindungen enthält.

6. Verwendung von mindestens einem bicyclischen Imidazo-3-yl-amin gemäß Anspruch 1, 2 oder 3 zusammen mit einem oder mehreren Hilfsstoffen zur Herstellung eines Arneimittels zur Bekämpfung von Schmerz.

7. Verfahren zur Herstellung von bicyclischen Imidazo-3-yl-aminen gemäß Anspruch 1, 2 oder 3 durch Dreikomponentenreaktion aus Amidin, Aldehyd und Isonitril, **dadurch gekennzeichnet, daß** die Synthese der Verbindungen in Dichlormethan als. Lösungsmittel und in Gegenwart von Perchlorsäure erfolgt, wobei die Ausgangsverbindungen nacheinander in der Reihenfolge Amidin, Aldehyd und Isonitril zugegeben werden und die entstehenden Produkte gegebenenfalls anschließend mit einer Verbindung R²Hal oder einem Isocyanat R^{e}NCO umgesetzt werden.

## Claims

1. Bicyclic imidazo-3-yl-amines of the general formula I 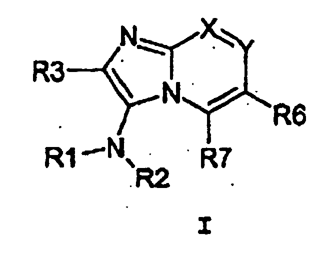 wherein
X denotes CR⁴ or N and Y denotes CR⁵ or N, with the proviso that X and Y do not simultaneously denote N,
R¹ denotes (CH₂)ₙCN, where n = 4, 5 or 6, optionally substituted phenyl, C₄-C₈-cycloalkyl, CH₂CH₂R (R = 4-morpholino), 1,1,3,3,-tetramethylbutyl or CH₂R^{a}, wherein R^{a} represents hydrogen, OH, C₁-C₈-alkyl (branched or unbranched), optionally substituted phenyl, CO(OR') (where R' = unbranched C₁-C₄-alkyl or branched C₁-C₅-alkyl), PO(OR')₂ (where R' = unbranched C₁-C₄-alkyl or branched C₁-C₅-alkyl) or Si(R^{x}R^{y}R^{z}) (where R^{x}, R^{y}, and R^{z} in each case independently of one another [sic] C₁-C₄-alkyl (branched or unbranched), C₄-C₈-cycloalkyl or phenyl),
R² denotes hydrogen, COR^{b}, wherein R^{b} represents C₁-C₄-alkyl (branched or unbranched) or C₃-C₈-cycloalkyl, CH₂CH₂CO(OR^{c}), wherein R^{c} represents C₁-C₄-alkyl (branched or unbranched), adamantyl, optionally substituted phenyl, optionally substituted 1-naphthyl or 2-naphthyl or in each case optionally substituted 2-pyridyl, 3-pyridyl, 4-pyridyl, thiazolyl or furoyl, CH₂phenyl, CH₂CH₂R^{d}, wherein R^{d} represents optionally substituted phenyl, or CONHR^{e}, wherein R^{e} represents C₁-C₈-alkyl (branched or unbranched), C₃-C₈-cycloalkyl or optionally substituted phenyl
R³ denotes C₁-C₈-alkyl (branched or unbranched) , C₃-C₈-cycloalkyl, optionally substituted phenyl, optionally substituted naphthyl, optionally substituted pyrrole, optionally substituted pyridyl, optionally substituted furan, optionally substituted thiophene, optionally substituted anthracene, optionally substituted phenanthrene or optionally substituted quinoline,
R⁴, R⁵, R⁶ and R⁷ are chosen from the group consisting of hydrogen, NO₂, NH₂, OH, CF₃, Cl, F, Br, CN, methyl or OR", wherein R" = benzyl, or R⁶ and R⁷ together form a bridge -CH=CH-CH=CH- and the radicals R⁴ and R⁵, if present, denote hydrogen,
with the proviso that at least one of the radicals R⁴, R⁵, R⁶ or R⁷ present in the molecule is not hydrogen, and if R¹ denotes methyl, ethyl, propyl, n-butyl, isobutyl, CO(Omethyl) or benzyl, R³ does not denote methyl, and if one of the radicals R⁴, R⁵, R⁶ and R⁷ denotes Obenzyl or R⁴ denotes OC₁-C₄-alkyl (branched or unbranched), R¹ does not denote CH₂R^{a} (wherein R^{a} represents hydrogen or C₁-C₅-alkyl (branched or unbranched), in the form of the bases or of pharmaceutically acceptable salts.

2. Bicyclic imidazo-3-yl-amines according to claim 1, **characterized in that** R² denotes hydrogen,
R¹ is chosen from the group consisting of (CH₂)ₙCH, where n = 4, 5 or 6, cyclohexyl, CH₂CO(Omethyl), 2,6-dimethylphenyl, 1,1,3,3,-tetramethylbutyl or n-butyl and
R³ is chosen from the group consisting of 2-pyridyl, 3-pyridyl, 2-furanyl, 2-pyrroyl, methyl, *tert*-butyl, 3-hydroxyphenyl, 3,4-dimethoxyphenyl, 2,3-dichlorophenyl, 2,4-dichlorophenyl, 2-methoxyphenyl, 2,3-dimethoxyphenyl, 3-bromophenyl, 4-bromo-2-fluorophenyl, 5-bromo-2-fluorophenyl, 3-bromo-4-fluorophenyl, 3-chlorophenyl, 3,4-dichlorophenyl, 3-fluorophenyl, 3-methylphenyl, 3-phenoxyphenyl,- 3-(4-chlorophenoxy)phenyl, 2-chloro-4-fluorophenyl, 2-chloro-6-fluorophenyl, 2,4-dimethylphenyl, 2,5-dimethylphenyl, 2-bromophenyl, 2-fluorophenyl, 2-(trifluoromethyl)-phenyl.

3. Bicyclic imidazo-3-yl-amines according to one of claims 1 to 2, **characterized in that** they are
7-chloro-2-furan-2-yl-(6-isocyanohexyl)-imidazo[1,2-a]pyrimidine-3,5-diamine,
(5,7-dimethyl-2-pyridin-2-yl-imidazo[1,2-a]pyrimidin-3-yl)-(6-isocyanohexyl)-amine,
7-chloro-(1,1,3,3-tetramethylbutyl)-2-thiophen-2-yl-imidazo[1,2-a]pyrimidine-3,5-diamine,
[6-bromo-2-(2-methoxyphenyl)-imidazo[1,2-a]pyridin-3-yl]-(1,1,3,3-tetramethylbutyl)-amine,
*N*-[4-(8-benzyloxy-3-cyclohexylamino-imidazo[1,2-a]pyridin-2-yl)-phenyl]-acetamide,
3-(8-benzyloxy-3-butylamino-imidazo[1,2-a]pyridin-2-yl)-phenol,
[8-benzyloxy-2-(3,5-dimethoxyphenyl)-imidazo[1,2-a]pyridin-3-ylamino]-acetic acid methyl ester,
[8-benzyloxy-2-(3,5-dimethoxyphenyl)imidazo[1,2-a]pyridin-3-yl]-cyclohexyl-amine,
cyclohexyl-[6,8-dibromo-2-(2-methoxyphenyl)-5-methyl-imidazo[1,2-a]pyridin-3-yl]-amine,
3-[3-(2,6-dimethylphenylamino)-6-nitro-imidazo[1,2-a]pyridin-2-yl-phenol,
[6-bromo-2-(2-methoxyphenyl)-imidazo[1,2-a]pyridin-3-yl]-(1,1,3,3-tetramethylbutyl)-amine,
[6,8-dibromo-2-(2,3-dimethoxyphenyl)-5-methyl-imidazo[1,2-a]pyridin-3-yl]-(1,1,3,3-tetramethylbutyl)-amine,
cyclohexyl-(2-phenyl-imidazo[1,2-a]quinolin-1-yl)-amine.
[6-(2-cyclohexyl-5-methyl-imidazo[1,2-a]pyridin-3-ylamino)-hexyl]-methylidyne-ammonium,
(2,6-dimethyl-imidazo[1,2-a]pyridin-3-yl)-(1,1,3,3-tetramethylbutyl)-amine,
cyclohexyl-(2,7-dimethyl-imidazo[1,2-a]pyridin-3-yl)-amine,
cyclohexyl-(2,5,7-trimethyl-imidazo[1,2-a]pyridin-3-yl)-amine,
[2-(3,4-dimethoxyphenyl)-6-methyl-imidazo[1,2-a]pyridin-3-yl]-(6-isocyanohexyl)-amine,
(2,7-dimethyl-imidazo[1,2-a]pyridin-3-yl)-(1,1,3,3-tetramethyl-butyl)-amine,
(2,8-dimethyl-imidazo[1,2-a]pyridin-3-yl)-(1,1,3,3-tetramethyl-butyl)-amine
(1,1,3,3-tetramethyl-butyl)-(2,5,7-trimethyl-imidazo[1,2-a]pyridin-3-yl)-amine,
[2-(3,4-dimethoxy-phenyl)-7-methyl-imidazo[1,2-a]pyridin-3-yl]-(6-isocyano-hexyl)-amine,
(6-isocyano-hexyl)-[2-(2-methoxy-phenyl)-6-methyl-imidazo[1,2-a]pyridin-3-yl]-amine,
cyclohexyl-(2-furan-2-yl-6-trifluoromethyl-imidazo[1,2-a]pyridin-3-yl)-amine;
(8-benzyloxy-2-cyclohexyl-imidazo[1,2-a]pyridin-3-yl)-cyclohexyl-amine,
(8-benzyloxy-2-methyl-imidazo[1,2-a]pyridin-3-yl)-cyclohexyl-amine,
(8-benzyloxy-2-methyl-imidazo[1,2-a]pyridin-3-yl)-(1,1,3,3-tetramethyl-butyl)-amine,
(8-benzyloxy-2-cyclohexyl-imidazo[1,2-a]pyridin-3-ylamino)-acetic acid methyl ester,
(8-benzyloxy-2-methyl-imidazo[1,2-a]pyridin-3-ylamino)-acetic acid methyl ester,
butyl-(2-cyclohexyl-5-propyl-imidazo[1,2-a]pyridin-3-yl)-amine,
N-cyclohexyl-N-(6,8-dichloro-2-furan-2-yl-imidazo[1,2-a]pyridin-3-yl)-acetamide,
N-cyclohexyl-N-(2-furan-2-yl-6-trifluoromethyl-imidazo[1,2-a]pyridin-3-yl)-acetamide,
N-(8-benzyloxy-2-cyclohexyl-imidazo[1,2-a]pyridin-3-yl)-N-cyclohexyl-acetamide,
(5-methyl-2-phenanthren-9-yl-imidazo[1,2-a]pyridin-3-yl)-(1,1,3,3-tetramethyl-butyl)-amine,
(2-anthracen-9-yl-7-methyl-imidazo[1,2-a]pyrimidin-3-yl)-(1,1,3,3-tetramethyl-butyl)-amine or
cyclohexyl-[7-methyl-2-(1-methyl-1H-pyrrol-2-yl)-imidazo[1,2-a]pyrimidin-3-yl]-amine.

4. Medicaments comprising as the active compound at least one bicyclic imidazo-3-yl-amine of the general formula 1 according to claim 1, in which R¹ to R⁷, X and Y have the meaning given in claim 1, in the form of the free base or a pharmaceutically acceptable salt.

5. Medicament according to claim 4, **characterized in that** it comprises as the active compound at least one bicyclic imidazo-3-yl-amine chosen from the group consisting of
7-chloro-2-furan-2-yl-(6-isocyano-hexyl)-imidazo[1,2-a]pyrimidine-3,5-diamine,
(5,7-dimethyl-2-pyridin-2-yl-imidazo[1,2-a]pyrimidin-3-yl)-(6-isocyanohexyl)-amine,
7-chloro-(1,1,3,3-tetramethylbutyl)-2-thiophen-2-yl-imidazo[1,2-a]pyrimidine-3,5-diamine,
[6-bromo-2-(2-methoxyphenyl)-imidazo[1,2-a]pyridin-3-yl]-(1,1,3,3-tetramethylbutyl)-amine,
*N*-[4-(8-benzyloxy-3-cyclohexylamino-imidazo[1,2-a]pyridin-2-yl)-phenyl]-acetamide,
3-(8-benzyloxy-3-butylamino-imidazo[1,2-a]pyridin-2-yl)-phenol,
[8-benzyloxy-2-(3,5-dimethoxyphenyl)-imidazo[1,2-a]pyridin-3-ylamino]-acetic acid methyl ester,
[8-benzyloxy-2-(3,5-dimethoxy-phenyl)-imidazo[1,2-a]pyridin-3-yl]-cyclohexyl-amine,
cyclohexyl-[6,8-dibromo-2-(2-methoxyphenyl)-5-methyl-imidazo[1,2-a]pyridin-3-yl]-amine,
3-[3-(2,6-dimethylphenylamino)-6-nitro-imidazo[1,2-a]pyridin-2-yl-phenol,
[6-bromo-2-(2-methoxyphenyl)-imidazo[1,2-a]pyridin-3-yl]-(1,1,3,3-tetramethylbutyl)-amine, [6,8-dibromo-2-(2,3-dimethoxyphenyl)-5-methyl-imidazo[1,2-a]pyridin-3-yl]-(1,1,3,3-tetramethylbutyl)-amine or
cyclohexyl-(2-phenyl-imidazo[1,2-a]quinolin-1-yl)-amine
[6-(2-cyclohexyl-5-methyl-imidazo[1,2-a]pyridin-3-ylamino)-hexyl]-methylidyne-ammonium,
(2,6-dimethyl-imidazo[1,2-a]pyridin-3-yl)-(1,1,3,3-tetramethylbutyl)-amine,
cyclohexyl-(2,7-dimethyl-imidazo[1,2-a]pyridin-3-yl)-amine,
cyclohexyl-(2,5,7-trimethyl-imidazo[1,2-a]pyridin-3-yl)-amine,
[2-(3,4-dimethoxyphenyl)-6-methyl-imidazo[1,2-a]pyridin-3-yl]-(6-isocyanohexyl)-amine, (2,7-dimethyl-imidazo[1,2-a]pyridin-3-yl)-(1,1,3,3-tetramethyl-butyl)-amine,
(2,8-dimethyl-imidazo[1,2-a]pyridin-3-yl)-(1,1,3,3-tetramethyl-butyl)-amine
(1,1,3,3-tetramethyl-butyl)-(2,5,7-trimethyl-imidazo[1,2-a]pyridin-3-yl)-amine
[2-(3,4-dimethoxy-phenyl)-7-methyl-imidazo[1,2-a]pyridin-3-yl]-(6-isocyano-hexyl)-amine,
(6-isocyano-hexyl)-[2-(2-methoxy-phenyl)-6-methyl-imidazo[1,2-a]pyridin-3-yl]-amine,
cyclohexyl-(2-furan-2-yl-6-trifluoromethyl-imidazo[1,2-a]pyridin-3-yl)-amine;
(8-benzyloxy-2-cyclohexyl-imidazo[1,2-a]pyridin-3-yl)-cyclohexyl-amine,
(8-benzyloxy-2-methyl-imidazo[1,2-a]pyridin-3-yl)-cyclohexyl-amine,
(8-benzyloxy-2-methyl-imidazo[1,2-a]pyridin-3-yl)-(1,1,3,3-tetramethyl-butyl)-amine, (8-benzyloxy-2-cyclohexyl-imidazo[1,2-a]pyridin-3-ylamino)-acetic acid methyl ester,
(8-benzyloxy-2-methyl-imidazo[1,2-a]pyridin-3-ylamino)-acetic acid methyl ester,
butyl-(2-cyclohexyl-5-propyl-imidazo[1,2-a]pyridin-3-yl)-amine,
N-cyclohexyl-N-(6,8-dichloro-2-furan-2-yl-imidazo[1,2-a]pyridin-3-yl)-acetamide,
N-cyclohexyl-N-(2-furan-2-yl-6-trifluoromethyl-imidazo[1,2-a]pyridin-3-yl)-acetamide,
N-(8-benzyloxy-2-cyclohexyl-imidazo[1,2-a]pyridin-3-yl)-N-cyclohexyl-acetamide,
(5-methyl-2-phenanthren-9-yl-imidazo[1,2-a]pyridin-3-yl)-(1,1,3,3-tetramethyl-butyl)-amine,
(2-anthracen-9-yl-7-methyl-imidazo[1,2-a]pyrimidin-3-yl)-(1,1,3,3-tetramethyl-butyl)-amine,
cyclohexyl-[7-methyl-2-(1-methyl-1H-pyrrol-2-yl)-imidazo[1,2-a]pyrimidin-3-yl]-amine or the pharmaceutically acceptable salts of these compounds.

6. Use of at least one bicyclic imidazo-3-yl-amine according to claim 1, 2 or 3 together with one or more auxiliary substances for the preparation of a medicament for combating pain.

7. Process for the preparation of bicyclic imidazo-3-yl-amines according to claim 1, 2 or 3 by a three-component reaction from amidine, aldehyde and isonitrile, **characterized in that** the synthesis of the compounds is carried out in methylene chloride as the solvent and in the presence of perchloric acid, the starting compounds being added successively in the sequence of amidine, aldehyde and isonitrile and the products formed optionally subsequently being reacted with a compound R²Hal or an isocyanate R^{e}NCO.

## Revendications

1. Imidazo-3-ylamines bicycliques de formule générale I 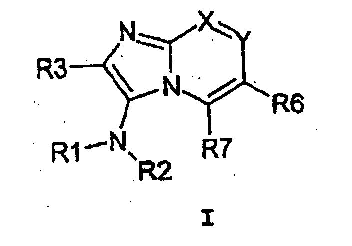 dans laquelle
X représente CR⁴ ou N et Y représente CR⁵ ou N, sous réserve que X et Y ne représentent pas N en même temps,
R¹ est un groupe (CH₂)ₙCN dans lequel n a la valeur 4, 5 ou 6, un reste phényle éventuellement substitué, cycloalkyle en C₄ à C₈, CH₂CH₂R (R = 4-morpholino), 1, 1, 3, 3-tétraméthylbutyle ou un groupe CH₂R^{a}, dans lequel R^{a} est l'hydrogène, un radical OH, alkyle en C₁ à C₈ (ramifié ou non ramifié), phényle éventuellement substitué, CO(OR') (avec R' = alkyle en C₁ à C₄ non ramifié ou alkyle en C₁ à C₅ ramifié), PO(OR')₂ (avec R' = alkyle en C₁ à C₄ non ramifié ou alkyle en C₁ à C₅ ramifié), ou bien Si (R^{x}R^{y}R^{z}) (avec R^{x}, R^{y} et R^{z} représentant chacun indépendamment l'un de l'autre un radical alkyle en C₁ à C₄ (ramifié ou non ramifié), cycloalkyle en C₄ à C₈ ou phényle),
R² représente l'hydrogène, un groupe COR^{b}, dans lequel R^{b} est un radical alkyle en C₁ à C₄ (ramifié ou non ramifié) ou cycloalkyle en C₃ à C₈, CH₂CH₂CO(OR^{c}), où R^{c} est un radical alkyle en C₁ à C₄ (ramifié ou non ramifié), adamantyle, phényle éventuellement substitué, 1-naphtyle ou 2-naphtyle éventuellement substitué, ou bien 2-pyridyle, 3-pyridyle, 4-pyridyle, thiazolyle ou furoyle dont chacun est éventuellement substitué, CH₂-phényle, CH₂CH₂R^{d}, où R^{d} est un reste phényle éventuellement substitué, ou CONHR^{e}, où R^{e} est un radical alkyle en C₁ à C₈ (ramifié ou non ramifié), cycloalkyle en C₃ à C₈ ou phényle éventuellement substitué,
R³ est un groupe alkyle en C₁ à C₈ (ramifié ou non ramifié), cycloalkyle en C₃ à C₈, phényle éventuellement substitué, naphtyle éventuellement substitué, pyrrole éventuellement substitué, pyridyle éventuellement substitué, furanne éventuellement substitué, thiophène éventuellement substitué, anthracène éventuellement substitué, phénanthrène éventuellement substitué ou quinoléine éventuellement substituée,
R⁴, R⁵, R⁶ et R⁷ sont choisis dans le groupe comprenant l'hydrogène, NO₂, NH₂, OH, CF₃, Cl, F, Br, CN, méthyle, ou OR" avec R"=benzyle ou R⁶ et R⁷ forment conjointement un pont -CH=CH-CH=CH- et les restes R⁴ et R⁵, dans la mesure où ils sont présents, désignent l'hydrogène,
sous réserve que l'un au moins des restes R⁴, R⁵, R⁶ et R⁷ présents dans la molécule ne soit pas de l'hydrogène et que, lorsque R¹ est un groupe méthyle, éthyle, propyle, n-butyle, isobutyle, CO(Ométhyle) ou benzyle, R³ ne soit pas un groupe méthyle, et que lorsque l'un des restes R⁴, R⁵, R⁶ et R⁷ désigne un reste Obenzyle ou bien R⁴ désigne un reste O(alkyle en C₁ à C₄) (ramifié ou non ramifié), R¹ ne représente pas un groupe CH₂R^{a} (dans lequel R^{a} est l'hydrogène ou un radical alkyle en C₁ à C₅ (ramifié ou non ramifié)), sous forme de bases ou de sels acceptables du point de vue pharmaceutique.

2. Imidazo-3-ylamines bicycliques suivant la revendication 1, **caractérisées en ce que** R² représente l'hydrogène,
R¹ est choisi dans le groupe des restes (CH₂)ₙCN avec n = 4, 5 ou 6, cyclohexyle, CH₂CO(Ométhyle), 2,6-diméthylphényle, 1,1,3,3-tétraméthylbutyle ou n-butyle et
R³ est choisi dans le groupe des restes 2-pyridyle, 3-pyridyle, 2-furannyle, 2-pyrroyle, méthyle, tertiobutyle, 3-hydroxyphényle, 3,4-diméthoxyphényle, 2,3-dichlorophényle, 2,4-dichlorophényle, 2-méthoxyphényle, 2,3-diméthoxyphényle, 3-bromophényle, 4-bromo-2-fluorophényle, 5-bromo-2-fluorophényle, 3-bromo-4-fluorophényle, 3-chlorophényle, 3,4-dichlorophényle, 3-fluorophényle, 3-méthylphényle, 3-phénoxyphényle, 3-(4-chlorophénoxy)-phényle, 2-chloro-4-fluorophényle, 2-chloro-6-fluorophényle, 2,4-diméthylphényle, 2,5-diméthylphényle, 2-bromophényle, 2-fluorophényle, 2-(trifluorométhyl)phényle.

3. Imidazo-3-ylamines bicycliques suivant l'une des revendications 1 et 2, **caractérisées en ce qu'**il s'agit de :
7-chloro-2-furanne-2-yl-(6-isocyanohexyl)imidazo-[1,2-a]pyrimidine-3,5-diamine,
(5,7-diméthyl-2-pyridine-2-ylimidazo-[1,2-a]pyrimidine-3-yl)-(6-isocyanohexyl)amine,
7-chloro-[1,1,3,3-tétraméthylbutyl)-2-thiophène-2-ylimidazo[1,2-a]pyrimidine-3,5-diamine,
[6-bromo-2-(2-méthoxyphényl)imidazo[1,2-a]pyridine-3-yl]-(1,1,3,3-tétraméthylbutyl)amine,
*N*-[4-(8-benzyloxy-3-cyclohexylamino-imidazo[1,2-a]pyridine2-yl)phényl]acétamide,
3-(8-benzyloxy-3-butylamino-imidazo[1,2-a]pyridine-2-yl)-phénol,
ester méthylique d'acide [8-benzyloxy-2-(3,5-diméthoxyphényl)imidazo[1,2-a]pyridine-3-ylamino]acétique,
[8-benzyloxy-2-(3,5-diméthoxyphényl)imidazo[1,2-a]pyridine-3-yl)cyclohexylamine,
cyclohexyl-[6,8-dibromo-2-(2-méthoxyphényl)-5-méthylimidazo[1,2-a]pyridine-3-yl]amine,
3-[3-(2,6-diméthylphénylamino)-6-nitro-imidazo[1,2-a]pyridine-2-yl]phénol,
[6-bromo-2-(2-méthoxyphényl)imidazo[1,2-a]pyridine-3-yl]-(1,1,3,3-tétraméthylbutyl)amine,
[6,8-dibromo-2-(2,3-diméthoxyphényl)-5-méthylimidazo[1,2-a]pyridine-3-yl]-(1,1,3,3-tétraméthylbutyl)amine,
cyclohexyl-(2-phénylimidazo[1,2-a]quinoléine-1-yl)amine.
[6-(2-cyclohexyl-5-méthylimidazo[1,2-a]pyridine-3-ylamino)-hexyl]méthylidène-ammonium,
(2,6-diméthylimidazo[1,2-a]pyridine-3-yl)-(1,1,3,3-tétraméthylbutyl)amine,
cyclohexyl-(2,7-diméthylimidazo[1,2-a]pyridine-3-yl)amine, cyclohexyl-(2,5,7-triméthylimidazo[1,2-a]pyridine-3-yl)-amine,
[2-(3,4-diméthoxyphényl)-6-méthylimidazo[1,2-a]pyridine-3-yl]-(6-isocyanohexyl)amine,
(2,7-diméthylimidazo[1,2-a]pyridine-3-yl)-(1,1,3,3-tétraméthylbutyl)amine,
(2,8-diméthylimidazo[1,2-a]pyridine-3-yl)-(1,1,3,3-tétraméthylbutyl)amine,
(1,1,3,3-tétraméthylbutyl)-(2,5,7-triméthylimidazo[1,2-a]pyridine-3-yl)amine,
[2-(3,4-diméthoxyphényl)-7-méthylimidazo[1,2-a]pyridine-3-yl]-(6-isocyanohexyl)amine,
(6-isocyanohexyl)-[2-(2-méthoxyphényl)-6-méthylimidazo[1,2-a]pyridine-3-yl]amine,
cyclohexyl-(2-furanne-2-yl-6-trifluorométhylimidazo[1,2-a]pyridine-3-yl)amine,
(8-benzyloxy-2-cyclohexylimidazo[1,2-a]pyridine-3-yl)cyclohexylamine,
(8-benzyloxy-2-méthylimidazo[1,2-a]pyridine-3-yl)cyclohexylamine,
(8-benzyloxy-2-méthylimidazo[1,2-a]pyridine-3-yl)-(1,1,3,3-tétraméthylbutyl)amine,
ester méthylique d'acide [8-benzyloxy-2-cyclohexylimidazo-[1,2-a]pyridine-3-ylamino]acétique,
ester méthylique d'acide [8-benzyloxy-2-méthylimidazo[1,2-a]pyridine-3-ylamino]acétique,
butyl-(2-cyclohexyl-5-propylimidazo[1,2-a]pyridine-3-yl)-amine
N-cyclohexyl-N-(6,8-dichloro-2-furanne-2-ylimidazo[1,2-a]-pyridine-3-yl)acétamide,
N-cyclohexyl-N-(2-furanne-2-yl-6-trifluorométhylimidazo-[1,2-a]pyridine-3-yl)acétamide,
N-(8-benzyloxy-2-cyclohexylimidazo[1,2-a]pyridine-3-yl)-N-cyclohexylacétamide,
(5-méthyl-2-phénanthrène-9-ylimidazo[1,2-a]pyridine-3-yl)-(1,1,3,3-tétraméthylbutyl)amine,
(2-anthracène-9-yl-7-méthylimidazo[1,2-a]pyrimidine-3-yl)-(1,1,3,3-tétraméthylbutyl)amine ou
cyclohexyl-[7-méthyl-2-(1-méthyl-1H-pyrrole-2-yl)imidazo-[1,2-a]pyrimidine-3-yl]amine.

4. Médicament contenant comme substance active au moins une imidazo-3-ylamine bicyclique de formule générale I suivant la revendication 1, dans laquelle R¹ à R⁷, X et Y ont la définition indiquée dans la revendication 1, sous forme de la base libre ou d'un sel pharmaceutiquement acceptable.

5. Médicament suivant la revendication 4, **caractérisé en ce qu'**il contient comme substance active au moins une imidazo-3-ylamine bicyclique choisie dans le groupe :
7-chloro-2-furanne-2-yl-(6-isocyanohexyl)imidazo-[1,2-a]pyrimidine-3,5-diamine,
(5,7-diméthyl-2-pyridine-2-ylimidazo-[1,2-a]pyrimidine-3-yl)-(6-isocyanohexyl)amine,
7-chloro-(1,1,3,3-tétraméthylbutyl)-2-thiophène-2-ylimidazo[1,2-a]pyrimidine-3,5-diamine,
[6-bromo-2-(2-méthoxyphényl)imidazo[1,2-a]pyridine-3-yl]-(1,1,3,3-tétraméthylbutyl)amine,
*N*-[4-(8-benzyloxy-3-cyclohexylamino-imidazo[1,2-a]pyridine-2-yl)phényl]acétamide,
3-(8-benzyloxy-3-butylamino-imidazo[1,2-a]pyridine-2-yl)-phénol,
ester méthylique d'acide [8-benzyloxy-2-(3,5-diméthoxyphényl)imidazo[1,2-a]pyridine-3-ylamino]acétique,
[8-benzyloxy-2-(3,5-diméthoxyphényl)imidazo[1,2-a]pyridine-3-yl)cyclohexylamine,
cyclohexyl-[6,8-dibromo-2-(2-méthoxyphényl)-5-méthylimidazo[1,2-a]pyridine-3-yl]amine,
3-[3-(2,6-diméthylphénylamino)-6-nitro-imidazo[1,2-a]pyridine-2-yl)phénol,
[6-bromo-2-(2-méthoxyphényl)imidazo[1,2-a]pyridine-3-yl]-(1,1,3,3-tétraméthylbutyl)amine,
[6,8-dibromo-2-(2,3-diméthoxyphényl)-5-méthylimidazo[1,2-a]pyridine-3-yl]-(1,1,3,3-tétraméthylbutyl)amine ou cyclohexyl-(2-phénylimidazo[1,2-a]quinoléine-1-yl)amine,
[6-(2-cyclohexyl-5-méthylimidazo[1,2-a]pyridine-3-ylamino)-hexyl)méthylidène-ammonium,
(2,6-diméthylimidazo[1,2-a]pyridine-3-yl)-(1,1,3,3-tétraméthylbutyl)amine,
cyclohexyl-(2,7-diméthylimidazo[1,2-a]pyridine-3-yl)amine, cyclohexyl-(2,5,7-triméthylimidazo[1,2-a]pyridine-3-yl)-amine,
[2-(3,4-diméthoxyphényl)-6-méthylimidazo[1,2-a]pyridine-3-yl]-(6-isocyanohexyl)amine,
(2,7-diméthylimidazo[1,2-a]pyridine-3-yl)-(1,1,3,3-tétraméthylbutyl)amine,
(2,8-diméthylimidazo[1,2-a]pyridine-3-yl)-(1,1,3,3-tétraméthylbutyl)amine,
(1,1,3,3-tétraméthylbutyl)-(2,5,7-triméthylimidazo[1,2-a]-pyridine-3-yl)amine,
[2-(3,4-diméthoxyphényl)-7-méthylimidazo[1,2-a]pyridine-3-yl]-(6-isocyanohexyl)amine,
(6-isocyanohexyl)-[2-(2-méthoxyphényl)-6-méthylimidazo-[1,2-a]pyridine-3-yl)amine,
cyclohexyl-(2-furanne-2-yl-6-trifluorométhylimidazo[1,2-a]pyridine-3-yl)amine,
(8-benzyloxy-2 -cyclohexylimidazo[1,2-a]pyridine-3-yl)cyclohexylamine,
(8-benzyloxy-2-méthylimidazo[1,2-a]pyridine-3-yl)cyclohexylamine,
(8-benzyloxy-2-méthylimidazo[1,2-a]pyridine-3-yl)-(1,1,3,3-tétraméthylbutyl)amine,
ester méthylique d'acide [8-benzyloxy-2-cyclohexylimidazo-[1,2-a]pyridine-3-ylamino)acétique,
ester méthylique d'acide [8-benzyloxy-2-méthylimidazo[1,2-a]pyridine-3-ylamino]acétique, butyl-(2-cyclohexyl-5-propylimidazo[1,2-a]pyridine-3-yl)-amine,
N-cyclohexyl-N-(6,8-dichloro-2-furanne-2-ylimidazo[1,2-a]-pyridine-3-yl)acétamide,
N-cyclohexyl-N-(2-furanne-2-yl-6-trifluorométhylimidazo-[1,2-a]pyridine-3-yl)acétamide,
N-(8-benzyloxy-2-cyclohexylimidazo[1,2-a]pyridine-3-yl)-N-cyclohexylacétamide,
(5-méthyl-2-phénanthrène-9-ylimidazo[1,2-a]pyridine-3-yl)-(1,1,3,3-tétraméthylbutyl)amine,
(2-anthracène-9-yl-7-méthylimidazo[1,2-a]pyrimidine-3-yl)-(1,1,3,3-tétraméthylbutyl)amine ou
cyclohexyl-[7-méthyl-2-(1-méthyl-1H-pyrrole-2-yl)imidazo-[1,2-a]pyrimidine-3-yl]amine,
ou des sels pharmaceutiquement acceptables de ces composés.

6. Utilisation d'au moins une imidazo-3-ylamine bicyclique suivant la revendication 1, 2 ou 3 conjointement avec une ou plusieurs substances auxiliaires pour la préparation d'un médicament destiné à combattre la douleur.

7. Procédé de production d'imidazo-3-ylamines bicycliques suivant la revendication 1, 2 ou 3, par réaction à trois composants amidine, aldéhyde et isonitrile, **caractérisé en ce que** la synthèse des composés est conduite dans du dichlorométhane utilisé comme solvant et en présence d'acide perchlorique, les composés de départ étant ajoutés successivement dans l'ordre amidine, aldéhyde et isonitrile et les produits formés étant éventuellement amenés ensuite à réagir avec un composé R²Hal ou un isocyanate R^{e}NCO.
